# EUROPEAN PATENT SPECIFICATION

(11) **EP 2 364 706 B1**
(45) Date of publication and mention of the grant of the patent: **29.07.2015**
(21) Application number: 09830467.8
(22) Date of filing: 04.12.2009
(51) Int. Cl.: A61K 31/47, A61K 31/4709, A61K 31/496, A61K 31/5377, A61K 31/55, A61P 25/00, A61P 25/16, A61P 43/00, C07D 215/20, C07D 215/26, C07D 215/38, C07D 215/40, C07D 215/48, C07D 401/04, C07D 401/06, C07D 405/04, C07D 409/04, C07D 215/22

(54) **PHARMACEUTICAL AGENT COMPRISING QUINOLONE COMPOUND**
PHARMAZEUTISCHER WIRKSTOFF MIT CHINOLONVERBINDUNG
AGENT PHARMACEUTIQUE COMPRENANT UN COMPOSÉ DE QUINOLONE

(30) Priority: 05.12.2008 JP 2008310716
(43) Date of publication of application: 14.09.2011
(73) Proprietor: Otsuka Pharmaceutical Co., Ltd., Tokyo 101-8535 (JP)
(72) Inventor: OTSUBO, Kenji, Osaka-shi Osaka 541-0045 (JP); OCHI, Yuji, Osaka-shi Osaka 541-0045 (JP); NAKAI, Masami, Osaka-shi Osaka 541-0045 (JP); MORI, Atsushi, Osaka-shi Osaka 541-0045 (JP); MATSUZAKI, Takayuki, Osaka-shi Osaka 541-0045 (JP)
(74) Representative: Hoffmann Eitle
(86) International application number: PCT/JP2009/070383
(87) International publication number: WO 2010/064701

(56) References cited:
- WO-A1-98/17662
- WO-A1-2008/150029
- WO-A1-2009/053799
- WO-A2-01/12607
- WO-A2-02/22074
- WO-A2-99/32449
- JIN, G.H. ET AL.: 'Synthesis of azaisoflavones and their inhibitory activities of NO production in activated microglia' BIOORGANIC & MEDICINAL CHEMISTRY LETTERS vol. 18, no. 14, 13 June 2008, pages 4092 - 4094, XP022852904
- XIAO, Z. ET AL.: 'Synthesis, antiproliferative activity, and structure-activity relationships of 3-aryl-1H-quinolin-4-ones' CHEMMEDCHEM vol. 3, no. 7, 2008, pages 1077 - 1082, XP055032277
- FISKUM, G. ET AL.: ''Mitochondrial Mechanisms of Neural Cell Death and Neuroprotective Interventions in Perkinson's Disease'' ANN.N.Y. ACAD.SCI. vol. 991, 2003, pages 111 - 119
- KAZUTOSHI NAKANO ET AL.: 'Saiboshi o Miru Mitochondria to Saiboshi' IGAKU NO AYUMI vol. 225, no. 6, 10 May 2008, pages 501 - 506

## Description

### Technical Field

The present invention relates to a therapeutic and/or prophylactic agent for neurodegenerative diseases, diseases induced by neurological dysfunction, or diseases induced by deterioration of mitochondrial function, the agent comprising a quinolone compound or a salt thereof as an active ingredient.

### Background Art

Parkinson's disease is a chronic, progressive neurodegenerative disease that generally develops after middle age. Initial symptoms include unilateral resting tremor, akinesia and rigidity. The tremors, akinesia, and rigidity are called the three major signs of Parkinson's disease, and each of them is caused by the selective death of dopaminergic neurons projected from the substantia nigra to the striatum. The etiology of the disease is still unknown; however, accumulated evidence suggests that an impaired energy-generating system accompanied by abnormal mitochondrial function of nigrostriatal dopaminergic neurons triggers the neurodegenerative disorder of the disease. The mitochondrial dysfunction has been assumed to subsequently cause oxidative stress and failure of calcium homeostasis, thereby resulting in neurodegeneration (NPL 1).

Treatments of Parkinson's disease are roughly classified into medical management (medication) and surgical management (stereotaxic operation). Of these, medication is an established therapy and regarded as a basic treatment. In the medication, a symptomatic therapeutic agent is used to compensate for the nigrostriatal dopaminergic neuronal function denatured by Parkinson's disease. L-dopa exhibits the most remarkable therapeutic effects. It is said that no agent exceeds the effectiveness of L-dopa. Currently, L-dopa is used together with a dopa decarboxylase inhibitor to prevent the metabolism thereof in the periphery, and the desired clinical effects have been obtained.

However, L-dopa treatment has drawbacks in that, after several years of usage, there is a recurrence of movement disorders such as dyskinesia, and the sustainability and stability of the drug's effects are lost, resulting in fluctuations within each day. Moreover, side effects including digestive problems such as nausea and vomiting brought on by excessive release of dopamine, circulatory organ problems such as orthostatic hypotension, tachycardia and arrhythmia, and neurological manifestations such as hallucination, delusion and distraction have been a cause for concern.

Thus, in order to decrease the L-dopa preparation dosage and thereby reduce the side effects, multidrug therapies, in which dopamine receptor agonists, dopamine metabolism enzyme inhibitors, dopamine releasers, central anticholinergic agents and the like are used in combination, are employed. While such therapeutic advances remarkably improve prognoses, there is still no fundamental cure for Parkinsons disease and other neurodegenerative diseases. Medication must be taken for the rest of the patient's life, and the aforementioned drawbacks, i.e., decreased efficacy during long-term administration, side effects, and uncontrollable disease progression, can result from L-dopa monotherapy. In addition, it is difficult to expect dramatic effects, even with the employment of multidrug therapies.

Alzheimer's disease is a progressive neurodegenerative disease that affects various cognitive functions, primarily causing impairment of memory. Pathologically, Alzheimer's disease is characterized by the degeneration of synapses or neurons in the hippocampus and cerebral cortex, and the accumulation of two types of abnormal fibrils, i.e., senile plaques and changes in neurofibrils. Although the disease etiology is not completely understood, amyloid β protein (Aβ), which is derived from amyloid precursor protein (APP) by various mechanisms, is known to play an important role. Currently, cholinesterase inhibitors (tacrine, Aricept, rivastigmine, and galantamine) are used in the treatment of Alzheimer's disease for ameliorating symptoms, because acetylcholinergic nervous system in the brain is involved in cognitive function, and marked deficits in the acetylcholinergic system are observed in Alzheimer's disease. N-methyl-D-aspartate glutamate receptor antagonists (memantine) are also in practical use because hyperexcitability of the mechanism of glutamate neurotransmission is associated with neural degeneration or impairment. Neither monotherapy nor combination therapy using these drugs, however, has produced sufficient therapeutic effects, nor are they capable of halting the progression of the disease. Furthermore, gastrointestinal symptoms such as nausea and diarrhea are observed as side effects of cholinesterase.

With respect to ischemic neurodegenerative disorders induced by cerebral infarctions, such as atherothrombotic cerebral infarction, lacunar infarction, cardiogenic cerebral embolism, etc., the usage of very early thrombolytic therapy using tissue plasminogen activator (tPA) is rapidly increasing. This therapy, however, has many problems including a time window as short as within three hours after the onset of disease, hemorrhagic complications, etc.

In Japan, a free radical scavenger, edaravone, is used for a brain protection therapy. Although edaravone can be used concomitantly with tPA, sufficient clinical results have not been obtained.

Accordingly, there exists a strong need for a pharmaceutical agent having a novel mechanism of action, or a neuroprotectant for preventing neural degeneration or impairment from its etiologies such as abnormal mitochondrial function, etc.

PTL 1 discloses a quinolone compound or a salt thereof that is effective as an anticancer agent; however, PTL 1 does not teach that the compound or a salt thereof is effective as a therapeutic and/or prophylactic agent for neurodegenerative diseases, diseases induced by neurological dysfunction, or diseases induced by deterioration of mitochondrial function.

Additionally, PTL 2 discloses a quinolone compound that is effective for preventing intimal proliferation; however, PTL 2 but does not teach that the compound is effective as a therapeutic and/or prophylactic agent for neurodegenerative diseases, diseases induced by neurological dysfunction, or diseases induced by deterioration of mitochondrial function.

### Citation List

### Patent Literature

PTL 1: WO 2001/012607
PTL 2: WO 2002/022074

### Non Patent Literature

NPL 1: Ann. N.Y. Acad. Sci. 991: 111-119 (2003)
Jin et al., Bioorg. Med. Chem. Lett. vol. 18, no. 14, 13 June 2008, pages 4092-4094 describe the synthesis of azaisoflavones and examine their inhibitory activities of NO production in activated microglia. Said inhibitory activity is believed to be a mechanism for neuroprotective action.
WO 98/17662 A1 relates to the use of a azaisoflavones compound of a specific formula, having a hydroxyl group at position 5, in the treatment of certain diseases (in particular, diseases associated with cell proliferations) and the inhibition of protein kinases.
WO 2008/150029 (A1) is a post-published document pursuant to Article 54(3) EPC and describes a quinolone compound of a specific formula, which suppresses progression of neurological dysfunction by inhibiting the chronic progression of Parkinson's disease or protecting dopamine neurons from the disease etiology.
WO 2009/053799 is a post-published document pursuant to Article 54(3) EPC and concerns quinolone and cinnolinone compounds for use as cannabinoid receptor modulators, in particular cannabinoid 1 (CB1) or cannabinoid 2 (CB2) receptor modulators. The compounds are said to be useful for for treating diseases, conditions and/or disorders modulated by a cannabinoid receptor (such as pain, neurodegenrative disorders, eating disorders, weight loss or control, and obesity).
WO 99/32449 concerns compounds of a specific formula having a ring A condensed to a di-OR substituted benzene ring, wherein A is an orthocondensed heterocycle optionally substituted by (C1-4)alkyl, (C1-4)alkoxy or -COOR', and necessarily substituted by a -B-Cy group wherein B is methylene, ethylene, amino, CONH or a bond; and is a 5- or 6-membered heterocycle containing from 1 to 3 nitrogen atom(s) optionally substituted by one or more halogen(s). These compounds are PDE 4 and TNF alpha inhibitors.
Xiao et al., ChemMedChem Vol. 3, no. 7, 2008, pages 1077 - 1082 describe the synthesis, antiproliferative activity, and structure-activity relationships of 3-aryl-1H-quinolin-4-ones.
Kazutoshi Nakano et al., Igaku no Ayumi Vol. 225, no. 6, 10, pages 501 - 506, describe investigations about the role of mitochondria in cell death.

### Summary of Invention

### Technical Problem

An object of the present invention is to provide a therapeutic and/or prophylactic agent that inhibits the chronic progression of Parkinson's disease or protects dopamine neurons from the disease itself, thereby suppressing the progression of neurological dysfunction, so as to prolong the period of time until L-dopa is administered while also improving neuronal function.

Another object of the invention is to provide a pharmaceutical agent that is useful in treating diseases that induce cell death, and more specifically, to provide a pharmaceutical agent having efficacy for treating Alzheimer's disease, or improving dysfunction or neurologic deficits induced by cerebral apoplexy.

### Solution to Problem

The present inventors conducted extensive research to accomplish the aforementioned object. Consequently, they succeeded in producing a compound represented by Formula (1) shown below, which protects and improves mitochondrial function, and/or protects neurons and repairs neuronal function. The present invention has been accomplished based on the above findings.

The invention provides a therapeutic and/or prophylactic agent comprising a quinolone compound and a method for treating and/or preventing diseases as set forth in the following Items 1 to 4.
[Item 1] A quinolone compound represented by Formula (1) for use in the therapy and/or prophylaxis of neurodegenerative diseases, diseases induced by neurological dysfunction, or diseases induced by deterioration of mitochondrial function: 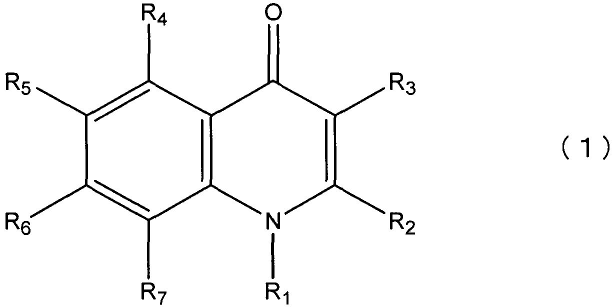 or a salt thereof, wherein:
   R₁ represents hydrogen;
   R₂ represents hydrogen or C₁-C₆ alkyl; and
   R₃ represents phenyl, naphthyl, pyridyl, furyl, thienyl, indolyl, benzodioxolyl or benzothienyl, wherein the aromatic or heterocyclic ring represented by R₃ may be substituted with one or more substituents selected from the group consisting of the following substituents (1) to (6):
      (1) C₁-C₆ alkyl,
      (2) halogen-substituted C₁-C₆ alkyl,
      (3) C₁-C₆ alkoxy,
      (4) halogen-substituted C₁-C₆ alkoxy,
      (5) phenyl optionally having one or more substituents selected from the group consisting of C₁-C₆ alkyl and C₁-C₆ alkoxy, and
      (6) halogen;
   R₄ represents hydrogen, C₁-C₆ alkyl, halogen-substituted C₁-C₆ alkyl, C₁-C₆ alkoxy, C₁-C₆ alkoxy C₁-C₆ alkyl, phenyl, cyclo C₃-C₈ alkyl, or carbamoyl optionally having one or two C₁-C₆ alkyl groups;
   R₅ represents hydrogen, C₁-C₆ alkyl, halogen, C₁-C₆ alkoxy, benzoylamino, or imidazolyl,
   R₆ represents hydrogen, halogen, C₁-C₆ alkyl, or C₁-C₆ alkoxy; and
   R₇ represents any of the following groups (1) to (17) :
      (1) C₁-C₆ alkyl,
      (2) C₁-C₆ alkoxy,
      (3) phenoxy,
      (4) cyclo C₃-C₈ alkyloxy,
      (5) halogen,
      (6) C₁-C₆ alkylthio,
      (7) amino optionally having one or two substituents selected from the group consisting of C₁-C₆ alkyl, C₁-C₆ alkoxy C₁-C₆ alkyl, and cyclo C₃-C₈ alkyl,
      (8) carbamoyl optionally having one or two C₁-C₆ alkyl groups,
      (9) pyrrolidinyl,
      (10) azepanyl,
      (11) morpholinyl,
      (12) piperazinyl optionally having one or two C₁-C₆ alkyl groups,
      (13) imidazolyl optionally having one or two C₁-C₆ alkyl groups,
      (14) furyl,
      (15) thienyl,
      (16) benzothienyl, and
      (17) pyrrolidinylcarbonyl.
[Item 2] The quinolone compound for use according to Item 1, wherein the neurodegenerative disease is selected from the group consisting of Parkinson's disease, Parkinson's syndrome, juvenile parkinsonism, striatonigral degeneration, progressive supranuclear palsy, pure akinesia, Alzheimer's disease, Pick's disease, prion disease, corticobasal degeneration, diffuse Lewy body disease, Huntington's disease, chorea-acanthocytosis, benign hereditary chorea, paroxysmal choreoathetosis, essential tremor, essential myoclonus, Gilles de la Tourette's syndrome, Rett's syndrome, degenerative ballism, dystonia musculorum deformans, athetosis, spasmodic torticollis, Meige syndrome, cerebral palsy, Wilson's disease, Segawa's disease, Hallervorden-Spatz syndrome, neuroaxonal dystrophy, pallidal atrophy, spinocerebellar degeneration, cerebral cortical atrophy, Holmes-type cerebellar atrophy, olivopontocerebellar atrophy, hereditary olivopontocerebellar atrophy, Joseph disease, dentatorubropallidoluysian atrophy, Gerstmann-Straussler-Scheinker disease, Friedreich's ataxia, Roussy-Levy syndrome, May-White syndrome, congenital cerebellar ataxia, hereditary episodic ataxia, ataxia telangiectasia, amyotrophic lateral sclerosis, progressive bulbar palsy, spinal progressive muscular atrophy, spinobulbar muscular atrophy, Werdnig-Hoffmann disease, Kugelberg-Welander disease, hereditary spastic paraparesis, syringomyelia, syringobulbia, Arnold-Chiari malformation, Stiff-man syndrome, Klippel-Feil syndrome, Fazio-Londe syndrome, lower myelopathy, Dandy-Walker syndrome, spina bifida, Sjogren-Larsson syndrome, radiation myelopathy, age-related macular degeneration, and cerebral apoplexy selected from the group consisting of cerebral infarction and cerebral hemorrhage and/or associated dysfunction or neurologic deficits.
[Item 3] The quinolone compound for use according to Item 1, wherein the disease induced by neurological dysfunction is selected from the group consisting of spinal cord injury, chemotherapy-induced neuropathy, diabetic neuropathy, radiation damage, and a demyelinating disease selected from the group consisting of multiple sclerosis, acute disseminated encephalomyelitis, transverse myelitis, progressive multifocal leukoencephalopathy, subacute sclerosing panencephalitis, chronic inflammatory demyelinating polyneuropathy, and Guillain-Barre syndrome.
[Item 4] The quinolone compound for use according to Item 1, wherein the disease induced by deterioration of mitochondrial function is selected from the group consisting of Pearson's syndrome, diabetes, deafness, malignant migraine, Leber's disease, MELAS, MERRF, MERRF/MELAS overlap syndrome, NARP, pure myopathy, mitochondrial cardiomyopathy, myopathy, dementia, gastrointestinal ataxia, acquired sideroblastic anemia, aminoglycoside-induced hearing loss, complex III deficiency due to inherited variants of cytochrome b, multiple symmetric lipomatosis, ataxia, myoclonus, retinopathy, MNGIE, ANT1 disease, Twinkle disease, POLG disease, recurrent myoglobinuria, SANDO, ARCO, complex I deficiency, complex II deficiency, optic nerve atrophy, fatal infantile complex IV deficiency, mitochondrial DNA deficiency syndrome, Leigh's encephalomyelopathy, chronic progressive external ophthalmoplegia syndrome (CPEO), Kearns-Sayre syndrome, encephalopathy, lactacidemia, myoglobinuria, drug-induced mitochondrial diseases, schizophrenia, major depression disorder, bipolar I disorder, bipolar II disorder, mixed episode, dysthymic disorders, atypical depression, seasonal affective disorders, postpartum depression, minor depression, recurrent brief depressive disorder, intractable depression, chronic depression, double depression, and acute renal failure.

Each group in Formula (1) is specifically described below.

The term "lower" refers to a group having 1 to 6 carbons (preferably 1 to 4 carbons), unless otherwise specified.

Examples of lower alkyl groups include straight or branched C₁₋₆ (preferably C₁₋₄) alkyl groups such as methyl, ethyl, n-propyl, isopropyl, n-butyl, isobutyl, tert-butyl, sec-butyl, n-pentyl, 1-ethylpropyl, isopentyl, neopentyl, n-hexyl, 1,2,2-trimethylpropyl, 3,3-dimethylbutyl, 2-ethylbutyl, isohexyl, 3-methylpentyl, etc.

Examples of cyclo C₃-C₈ alkyl groups include cyclopropyl, cyclobutyl, cyclopentyl, cyclohexyl, cycloheptyl, cyclooctyl, etc.

Examples of cyclo C₃-C₈ alkyl lower alkyl groups include the lower alkyl groups having one to three (preferably one) cyclo C₃-C₈ alkyl group(s) described above.

Examples of lower alkoxy groups include straight or branched C₁₋₆ (preferably C₁₋₄) alkoxy groups such as methoxy, ethoxy, n-propoxy, isopropoxy, n-butoxy, isobutoxy, tert-butoxy, sec-butoxy, n-pentyloxy, isopentyloxy, neopentyloxy, n-hexyloxy, isohexyloxy, 3-methylpentyloxy, etc.

Examples of lower alkoxy lower alkyl groups include the lower alkyl groups having one to three (preferably one) lower alkoxy group(s) described above.

Examples of halogen atoms include fluorine, chlorine, bromine, and iodine.

Examples of halogen-substituted lower alkyl groups include the lower alkyl groups having one to seven halogen atom(s), preferably one to three halogen atom(s). Examples thereof include fluoromethyl, difluoromethyl, trifluoromethyl, chloromethyl, dichloromethyl, trichloromethyl, bromomethyl, dibromomethyl, dichlorofluoromethyl, 2,2-difluoroethyl, 2,2,2-trifluoroethyl, pentafluoroethyl, 2-fluoroethyl, 2-chloroethyl, 3,3,3-trifluoropropyl, heptafluoropropyl, 2,2,3,3,3-pentafluoropropyl, heptafluoroisopropyl, 3-chloropropyl, 2-chloropropyl, 3-bromopropyl, 4,4,4-trifluorobutyl, 4,4,4,3,3-pentafluorobutyl, 4-chlorobutyl, 4-bromobutyl, 2-chlorobutyl, 5,5,5-trifluoropentyl, 5-chloropentyl, 6,6,6-trifluorohexyl, 6-chlorohexyl, perfluorohexyl, etc.

Examples of halogen-substituted lower alkoxy groups include the lower alkoxy groups having one to seven halogen atom(s), preferably one to three halogen atom(s). Examples thereof include fluoromethoxy, difluoromethoxy, trifluoromethoxy, chloromethoxy, dichloromethoxy, trichloromethoxy, bromomethoxy, dibromomethoxy, dichlorofluoromethoxy, 2,2,2-trifluoroethoxy, pentafluoroethoxy, 2-chloroethoxy, 3,3,3-trifluoropropoxy, heptafluoropropoxy, heptafluoroisopropoxy, 3-chloropropoxy, 2-chloropropoxy, 3-bromopropoxy, 4,4,4-trifluorobutoxy, 4,4,4,3,3-pentafluorobutoxy, 4-chlorobutoxy, 4-bromobutoxy, 2-chlorobutoxy, 5,5,5-trifluoropentoxy, 5-chloropentoxy, 6,6,6-trifluorohexyloxy, 6-chlorohexyloxy, etc.

Examples of lower alkylthio groups include alkylthio groups wherein the alkyl moiety is the lower alkyl group mentioned above.

Examples of phenyl groups optionally having one or more substituents selected from the group consisting of lower alkyl and lower alkoxy include phenyl groups optionally having one to three (preferably one or two) group(s) selected from the group consisting of the lower alkyl groups and the lower alkoxy groups described above.

Examples of carbamoyl groups optionally having one or more lower alkyl groups include carbamoyl groups optionally having one or two lower alkyl groups described above.

Examples of amino groups optionally having one or two substituents selected from the group consisting of lower alkyl groups, lower alkoxy lower alkyl groups, and cyclo C₃-C₈ alkyl groups include amino groups optionally having one or two groups selected from the group consisting of the lower alkyl groups, the lower alkoxy lower alkyl groups, and the cyclo C₃-C₈ alkyl groups described above.

Examples of piperazinyl groups optionally having one or two lower alkyl groups include piperazinyl groups optionally having one or two (preferably one) lower alkyl group(s) described above.

Examples of imidazolyl groups optionally having one or two lower alkyl groups include imidazolyl groups optionally having one or two (preferably one) lower alkyl group(s) described above.

Examples of lower alkoxy lower alkyl groups include the lower alkyl groups having one to three (preferably one) lower alkoxy group(s) described above.

Examples of cyclo C₃-C₈ alkyloxy groups include groups in which the cyclo C₃-C₈ alkyl groups described above are bonded to an oxygen atom.

The process of producing the compound of the invention is described below in detail.

The quinolone compound represented by Formula (1) (hereinafter also referred to as Compound (1)) can be produced by various methods; for example, by a method according to the following Reaction Scheme 1. 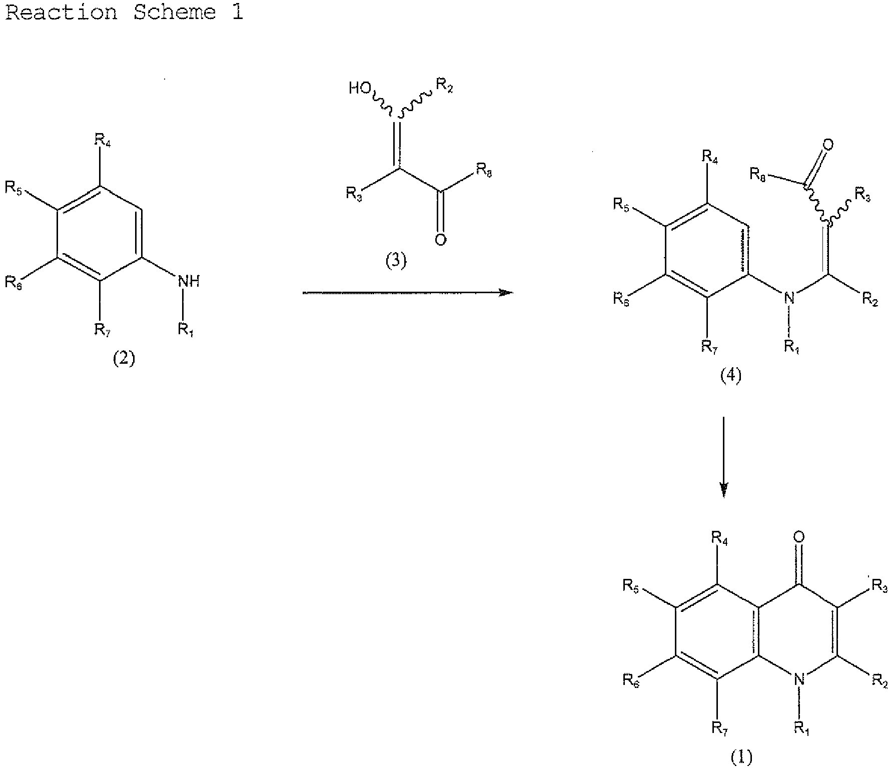 wherein R₁, R₂, R₃, R₄, R₅, R₆, and R₇ are as defined above, and R₈ represents a lower alkoxy group.

The lower alkoxy group represented by R₈ in Formula (3) has the same definition as described above.

The compound represented by Formula (2) is reacted with the compound represented by Formula (3) in an inert solvent or without using any solvents, in the presence or absence of an acid catalyst, thereby giving an intermediate compound represented by Formula (4). Then, the resulting compound is cyclized to produce the compound represented by Formula (1).

Examples of inert solvents include water; ethers such as dioxane, tetrahydrofuran, diethyl ether, 1,2-dimethoxyethane, diethylene glycol dimethyl ether, and ethylene glycol dimethyl ether; aromatic hydrocarbons such as benzene, toluene, and xylene; lower alcohols such as methanol, ethanol, and isopropanol; and polar solvents such as N,N-dimethylformamide (DMF), dimethyl sulfoxide (DMSO), hexamethylphosphoric triamide, and acetonitrile. These inert solvents can be used singly or in combinations of two or more.

Various kinds of known acid catalysts can be used, including toluenesulfonic acid, methanesulfonic acid, xylene sulfonic acid, sulfuric acid, glacial acetic acid, boron trifluoride, acidic ion exchangers, etc. These acid catalysts can be used singly or in combinations of two or more.

Among such acids, acidic ion exchangers are preferably used. Examples of acidic ion exchangers include polymeric cation exchangers available from the market such as Lewatit S100, Zeo-karb 225, Dowex 50, Amberlite IR120, or Amberlyst 15 and like styrene sulfonic acid polymers; Lewatit PN, Zeo-karb 215 or 315, and like polysulfonic acid condensates; Lewatit CNO, Duolite CS100, and like m-phenolic carboxylic acid resins; or Permutit C, Zeo-karb 226 or Amberlite IRC 50, and like polyacrylates. Of these, Amberlyst 15 is particularly preferred.

An acid catalyst is usually used in an amount of 0.0001 to 100 moles, preferably 0.5 to 6 moles, per mole of the compound of Formula (2).

In Reaction Scheme 1, the compound of Formula (3) is usually used in an amount of at least about 1 mole, preferably about 1 to about 5 moles, per mole of the compound of Formula (2) .

The reaction can be conducted under normal pressure, under inert gas atmospheres including nitrogen, argon, etc., or under increased pressure.

The reaction proceeds usually at room temperature to 200°C, and preferably at room temperature to 150°C. During the reaction, azeotropic removal of water is conducted until the reaction water generation is completed. The reaction is usually finished in about 1 to about 30 hours.

The process of producing the compound of Formula (1) via a cyclization reaction of the intermediate compound represented by Formula (4) can be carried out by heating the compound in a solvent such as diphenyl ether, or by heating the compound in the absence of a solvent. The reaction is conducted at 150 to 300°C for 5 minutes to 2 hours.

The compound represented by Formula (2), used as a starting material in Reaction Scheme 1, is a known compound or can be produced easily using a known compound. The compound represented by Formula (3) includes a novel compound, and the compound is manufactured in accordance with, for example, the method shown in Reaction Scheme 2 described below. 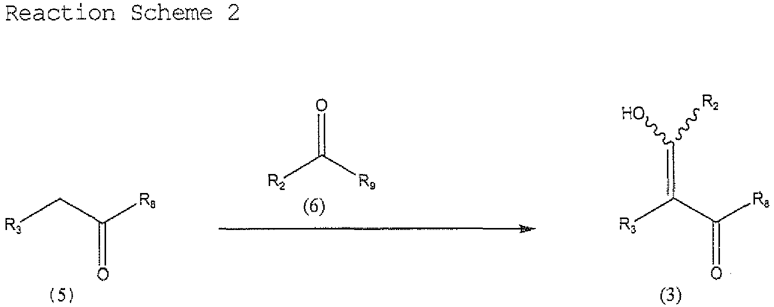 wherein R₂, R₃, and R₈ are as defined above, and R₉ represents a lower alkoxy group.

The lower alkoxy group represented by R₉ in Formula (6) has the same definition as described above.

The compound represented by Formula (3) can be produced by the reaction of the compound represented by Formula (5) with the compound represented by Formula (6) in an inert solvent or without using any solvents, in the presence or absence of a basic compound.

Examples of inert solvents include water; ethers such as dioxane, tetrahydrofuran, diethyl ether, 1,2-dimethoxyethane, diethylene glycol dimethyl ether, and ethylene glycol dimethyl ether; aromatic hydrocarbons such as benzene, toluene, and xylene; lower alcohols such as methanol, ethanol, and isopropanol; ketones such as acetone and methyl ethyl ketone; and polar solvents such as N,N-dimethylformamide (DMF), dimethyl sulfoxide (DMSO), hexamethylphosphoric triamide, and acetonitrile. These inert solvents can be used singly or in combinations of two or more.

As a basic compound, various known inorganic bases and organic bases can be used.

Inorganic bases include, for example, alkali metal hydroxides such as sodium hydroxide, potassium hydroxide, cesium hydroxide, and lithium hydroxide; alkali metal carbonates such as sodium carbonate, potassium carbonate, cesium carbonate, and lithium carbonate; alkali metal hydrogen carbonates such as lithium hydrogen carbonate, sodium hydrogen carbonate, and potassium hydrogen carbonate; alkali metals such as sodium and potassium; amides such as sodium amide; and alkali metal hydrides such as sodium hydride and potassium hydride.

Organic bases include, for example, alkali metal lower alkoxides such as sodium methoxide, sodium ethoxide, sodium t-butoxide, potassium methoxide, potassium ethoxide, and potassium t-butoxide; and amines such as triethylamine, tripropylamine, pyridine, quinoline, piperidine, imidazole, N-ethyl diisopropylamine, dimethylaminopyridine, trimethylamine, dimethylaniline, N-methylmorpholine, 1,5-diazabicyclo[4.3.0]non-5-ene (DBN), 1,8-diazabicyclo[5.4.0]undec-7-ene (DBU), 1,4-diazabicyclo[2.2.2]octane (DABCO), etc.

Such basic compounds can be used singly or in combinations of two or more. More preferable basic compounds used in the reaction include inorganic bases such as sodium hydride and potassium hydride.

A basic compound is usually used in an amount of 1 to 10 moles, preferably 1 to 6 moles, per mole of the compound of Formula (5).

In Reaction Scheme 2, the compound of Formula (6) is usually used in an amount of at least about 1 mole, preferably 1 to about 5 moles, per mole of the compound of Formula (5).

The reaction can be conducted under normal pressure, under inert gas atmospheres including nitrogen, argon, etc., or under increased pressure.

The reaction proceeds usually at room temperature to 200°C, and preferably at room temperature to 150°C, and is usually completed in about 1 to about 30 hours.

The compounds represented by Formulae (5) and (6), which are used as starting materials in Reaction Scheme 2, are easily available known compounds.

The raw material compounds used in each of the reaction schemes described above may include suitable salts, and the objective compounds obtained via each of the reactions may form suitable salts. These preferable salts include the following preferable salts of Compound (1).

Suitable salts of Compound (1) are pharmacologically allowable salts including, for example, salts of inorganic bases such as metal salts including alkali metal salts (e.g., sodium salts, potassium salts, etc.) and alkaline earth metal salts (e.g., calcium salts, magnesium salts, etc.), ammonium salts, alkali metal carbonates (e.g., lithium carbonate, potassium carbonate, sodium carbonate, cesium carbonate, etc.), alkali metal hydrogencarbonates (e.g., lithium hydrogencarbonate, sodium hydrogencarbonate, potassium hydrogencarbonate, etc.), and alkali metal hydroxides (e.g., lithium hydroxide, sodium hydroxide, potassium hydroxide, cesium hydroxide, etc.); salts of organic bases such as tri(lower)alkylamine (e.g., trimethylamine, triethylamine, N-ethyldiisopropylamine, etc.), pyridine, quinoline, piperidine, imidazole, picoline, dimethylaminopyridine, dimethylaniline, N-(lower)alkyl-morpholine (e.g., N-methylmorpholine, etc.), 1,5-diazabicyclo[4.3.0]non-5-ene (DBN), 1,8-diazabicyclo[5.4.0]undec-7-ene (DBU), and 1,4-diazabicyclo[2.2.2]octane (DABCO); inorganic acid salts such as hydrochloride, hydrobromide, hydroiodide, sulfate, nitrate, and phosphate; and organic acid salts such as formate, acetate, propionate, oxalate, malonate, succinate, fumarate, maleate, lactate, malate, citrate, tartrate, carbonate, picrate, methanesulfonate, ethanesulfonate, p-toluenesulfonate, and glutamate.

In addition, compounds in a form in which a solvate (for example, hydrate, ethanolate, etc.) was added to the starting materials and the objective compound shown in each of the reaction schemes are also included in each of the general formulae. Hydrate can be mentioned as a preferable solvate.

Each of the objective compounds obtained according to the above reaction schemes can be isolated and purified from the reaction mixture by, for example, cooling the reaction mixture, first, performing an isolation procedure such as filtration, concentration, extraction, etc., to separate a crude reaction product, and then subjecting the crude reaction product to a usual purification procedure such as column chromatography, recrystallization, etc.

The compound represented by Formula (1) according to the present invention naturally includes geometrical isomers, stereoisomers, optical isomers, and like isomers.

The following points should be noted regarding the compound of Formula (1) shown above. Specifically, when R₁ of Formula (1) represents a hydrogen atom, the compound includes a tautomer of the quinolone ring. That is, in the quinolone compound of Formula (1), when R₁ represents a hydrogen atom (1'), 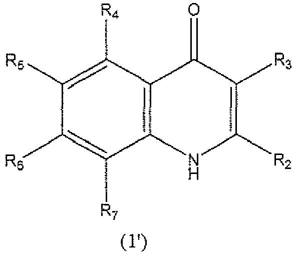 wherein R₂, R₃, R₄, R₅, R₆, and R₇ are as defined above, the compound of the tautomer can be represented by Formula (1"), 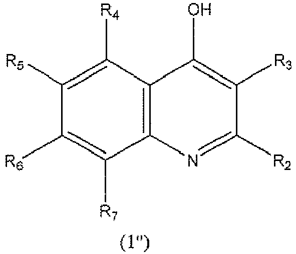 wherein R₂, R₃, R₄, R₅, R₆, and R₇ are as defined above. That is, both of the compounds represented by Formulae (1') and (1") are in the tautomeric equilibrium state represented by the following balance formula. 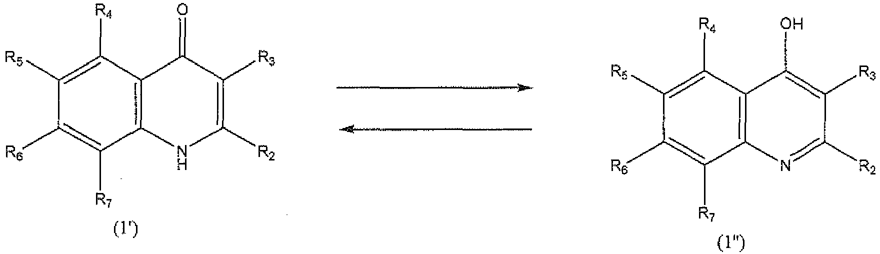 wherein R₂, R₃, R₄, R₅, R₆, and R₇ are as defined above. Such tautomerism between a 4-quinolone compound and a 4-hydroxyquinoline compound is technically known, and it is obvious for a person skilled in the art that both of the above-described tautomers are balanced and mutually exchangeable.

Therefore, the compound represented by Formula (1) of the present invention naturally includes the tautomers as mentioned above.

In the specification, the constitutional formula of a 4-quinolone compound is suitably used as a constitutional formula of the objective or starting material including compounds of such tautomers.

The present invention also includes isotopically labeled compounds that are identical to the compounds represented by Formula (1), except that one or more atoms are replaced by one or more atoms having specific atomic mass or mass numbers. Examples of isotopes that can be incorporated into the compounds of the present invention include hydrogen, carbon, nitrogen, oxygen, sulfur, fluorine, and chlorine, such as ²H, ³H, ¹³C, ¹⁴C, ¹⁵N, ¹⁸O, ¹⁷O, ¹⁸F, and ³⁶Cl. Certain isotopically labeled compounds of the present invention, which include the above-described isotopes and/or other isotopes of other atoms, for example, those into which radioisotopes such as ³H and ¹⁴C are incorporated, are useful in drug and/or substrate tissue distribution assay. Tritiated (i.e., ³H), and carbon-14 (i.e., ¹⁴C) isotopes are particularly preferred for their ease of preparation and detectability. Further, substitution with heavier isotopes such as deuterium (i.e., ²H) can afford certain therapeutic advantages resulting from greater metabolic stability, for example, an increased *in vivo* half-life or reduced dosage requirements. The isotopically labeled compounds of the present invention can generally be prepared by substituting a readily available, isotopically labeled reagent for a non-isotopically labeled reagent according to the method disclosed in the schemes above and/or in the Examples below.

The compound of Formula (1) and the salt thereof are used in the form of general pharmaceutical preparations. The preparations are obtained using typically employed diluents or excipients such as fillers, extenders, binders, wetting agents, disintegrators, surfactants, lubricants, etc. The form of such pharmaceutical preparations can be selected according to the purpose of the therapy. Typical examples include tablets, pills, powders, solutions, suspensions, emulsions, granules, capsules, suppositories, injections (solutions, suspensions, etc.), and the like.

To form tablets, any of various carriers conventionally known in this field can be used. Examples thereof include lactose, white sugar, sodium chloride, glucose, urea, starch, calcium carbonate, kaolin, crystalline cellulose, silicic acid, and other excipients; water, ethanol, propanol, simple syrup, glucose solutions, starch solutions, gelatin solutions, carboxymethylcellulose, shellac, methylcellulose, potassium phosphate, polyvinylpyrrolidone, and other binders; dry starch, sodium alginate, agar powder, laminarin powder, sodium hydrogen carbonate, calcium carbonate, fatty acid esters of polyoxyethylene sorbitan, sodium lauryl sulfate, stearic acid monoglycerides, starch, lactose, and other disintegrators; white sugar, stearin, cacao butter, hydrogenated oils, and other disintegration inhibitors; quaternary ammonium bases, sodium lauryl sulfate, and other absorption promoters; glycerol, starch, and other wetting agents; starch, lactose, kaolin, bentonite, colloidal silicic acid, and other adsorbents; purified talc, stearates, boric acid powder, polyethylene glycol, and other lubricants; etc. Further, such tablets may be coated with typical coating materials as required, to prepare, for example, sugar-coated tablets, gelatin-coated tablets, enteric-coated tablets, film-coated tablets, double- or multi-layered tablets, etc.

To form pills, any of various carriers conventionally known in this field can be used. Examples thereof include glucose, lactose, starch, cacao butter, hydrogenated vegetable oils, kaolin, talc, and other excipients; gum arabic powder, tragacanth powder, gelatin, ethanol, and other binders; laminarin, agar, and other disintegrators; etc.

To form suppositories, any of various carriers conventionally known in this field can be used. Examples thereof include polyethylene glycol, cacao butter, higher alcohols, esters of higher alcohols, gelatin, semi synthetic glycerides, etc.

Capsules can be prepared by mixing the active principal compound with the above-mentioned carriers to enclose the former in a hard gelatin capsule, soft gelatin capsule or the like.

To form an injection, a solution, emulsion or suspension is sterilized and preferably made isotonic to blood. Any of the diluents widely used for such forms in this field can be employed to form the injection. Examples of such diluents include water, ethyl alcohol, macrogol, propylene glycol, ethoxylated isostearyl alcohol, polyoxylated isostearyl alcohol, fatty acid esters of polyoxyethylene sorbitan, etc.

In this case, the pharmaceutical preparation may contain sodium chloride, glucose or glycerol in an amount sufficient to prepare an isotonic solution, and may contain typical solubilizers, buffers, analgesic agents, etc. Further, if necessary, the pharmaceutical preparation may contain coloring agents, preservatives, flavors, sweetening agents, etc., and/or other medicines.

The amount of the compound represented by Formula (1) and the salt thereof included in the pharmaceutical preparation of the present invention is not limited, and can be suitably selected from a wide range. The proportion is generally about 0.1 to about 70 wt.%, preferably about 0.1 to about 30 wt.% of the pharmaceutical preparation.

The route of administration of the pharmaceutical preparation of the present invention is not particularly limited, and the preparation is administered by a route suitable to the form of the preparation, patient's age, sex and other conditions, and severity of the disease. For example, tablets, pills, solutions, suspensions, emulsions, granules and capsules are administered orally. Injections are intravenously administered singly or as mixed with typical injection transfusions such as glucose solutions, amino acid solutions or the like, or singly administered intramuscularly, intracutaneously, subcutaneously or intraperitoneally, as required. Suppositories are administered intrarectally.

The dosage of the pharmaceutical preparation of the invention is suitably selected according to the method of use, patient's age, sex and other conditions, and severity of the disease. The amount of active principal compound is usually about 0.1 to about 10 mg/kg body weight/day. Further, it is desirable that the pharmaceutical preparation in each unit of the administration form contains the active principal compound in an amount of about 1 to about 200 mg.

The use of the compound of the present invention in combination with L-dopa preparations, dopamine receptor agonists, dopamine metabolism enzyme inhibitors, dopamine release-rate-promoting preparations, central anticholinergic agents, and the like can achieve effects such as dosage reduction, improvement of side effects, increased therapeutic efficacy, etc., which were not attained by known therapies.

### Advantageous Effect of Invention

The compound of the present invention protect and improve mitochondrial function and/or protect neurons and repair neuronal function, and hence are effective in the treatment and/or prevention of neurodegenerative diseases, diseases induced by neurological dysfunction, and diseases induced by deterioration of mitochondrial function.

Examples of neurodegenerative diseases include Parkinson's disease, Parkinson's syndrome, juvenile parkinsonism, striatonigral degeneration, progressive supranuclear palsy, pure akinesia, Alzheimer's disease, Pick's disease, prion disease, corticobasal degeneration, diffuse Lewy body disease, Huntington's disease, chorea-acanthocytosis, benign hereditary chorea, paroxysmal choreoathetosis, essential tremor, essential myoclonus, Gilles de la Tourette's syndrome, Rett syndrome, degenerative ballism, dystonia musculorum deformans, athetosis, spasmodic torticollis, Meige syndrome, cerebral palsy, Wilson's disease, Segawa's disease, Hallervorden-Spatz syndrome, neuroaxonal dystrophy, pallidal atrophy, spino-cerebellar degeneration, cerebral cortical atrophy, Holmes-type cerebellar atrophy, olivopontocerebellar atrophy, hereditary olivopontocerebellar atrophy, Joseph disease, dentatorubropallidoluysian atrophy, Gerstmann-Straussler-Scheinker disease, Friedreich's ataxia, Roussy-Levy syndrome, May-White syndrome, congenital cerebellar ataxia, hereditary episodic ataxia, ataxia telangiectasia, amyotrophic lateral sclerosis, progressive bulbar palsy, progressive spinal muscular atrophy, spinobulbar muscular atrophy, Werdnig-Hoffmann disease, Kugelberg-Welander disease, hereditary spastic paraparesis, syringomyelia, syringobulbia, Arnold-Chiari malformation, Stiff-man syndrome, Klippel-Feil syndrome, Fazio-Londe syndrome, lower myelopathy, Dandy-Walker syndrome, spina bifida, Sjogren-Larsson syndrome, radiation myelopathy, age-related macular degeneration, and cerebral apoplexy (e.g., cerebral infarction and cerebral hemorrhage) and/or dysfunction or neurologic deficits associated with cerebral apoplexy.

Examples of diseases induced by neurological dysfunction include spinal cord injury, chemotherapy-induced neuropathy, diabetic neuropathy, radiation damage, and demyelinating diseases (e.g., multiple sclerosis, acute disseminated encephalomyelitis, transverse myelitis, progressive multifocal leucoencephalopathy, subacute sclerosing panencephalitis, chronic inflammatory demyelinating polyneuropathy, and Guillain-Barre syndrome).

Examples of diseases induced by deterioration of mitochondrial function include Pearson's syndrome, diabetes, deafness, malignant migraine, Leber's disease, MELAS, MERRF, MERRF/MELAS overlap syndrome, NARP, pure myopathy, mitochondrial cardiomyopathy, myopathy, dementia, gastrointestinal ataxia, acquired sideroblastic anemia, aminoglycoside-induced hearing loss, complex III deficiency due to inherited variants of cytochrome b, multiple symmetrical lipomatosis, ataxia, myoclonus, retinopathy, MNGIE, ANT1 disease, Twinkle disease, POLG disease, recurrent myoglobinuria, SANDO, ARCO, complex I deficiency, complex II deficiency, optic nerve atrophy, fatal infantile complex IV deficiency, mitochondrial DNA deficiency syndrome, Leigh's encephalomyelopathy, chronic-progressive-externalophthalmoplegia syndrome (CPEO), Kearns-Sayre syndrome, encephalopathy, lactacidemia, myoglobinuria, drug-induced mitochondrial diseases, schizophrenia, major depression disorder, bipolar I disorder, bipolar II disorder, mixed episode, dysthymic disorders, atypical depression, seasonal affective disorders, postpartum depression, minor depression, recurrent brief depressive disorder, intractable depression, chronic depression, double depression, and acute renal failure.

The compound of the present invention can achieve effects heretofore unattained by known therapies, such as reduced dose, reduced side effects, and potentiated therapeutic effects, when it is administered in combination with L-dopa preparations, dopamine receptor agonists, dopamine metabolism enzyme inhibitors, dopamine release-rate-promoting preparations, central anticholinergic agents, cholinesterase inhibitors, N-methyl-D-aspartate glutamate receptor antagonists, or other agents used in thrombolytic therapy, cerebral edema therapy, brain protection therapy, antithrombotic therapy, and blood plasma dilution therapy.

### Description of Embodiments

Hereinafter, the present invention is described in more detail with reference to Reference Examples, Examples, and Pharmacological Test Examples.

### Reference Example 1

### 4-Methyl-2-nitro-1-propoxybenzene

A DMF solution (4 ml) of potassium carbonate (5.21 g, 37.7 mmol) and 1-iodopropane (5.80 g, 34.1 mmol) was added to a N,N-dimethylformamide (DMF) solution (10 ml) of 4-methyl-2-nitrophenol (4.0 g, 26.1 mmol), and the mixture was stirred at room temperature for 48 hours. Water was added to the reaction mixture, and the resulting mixture was extracted with ethyl acetate. The organic layer was washed with a saturated saline solution twice and concentrated under reduced pressure. The residue was purified using silica gel column chromatography (n-hexane:ethyl acetate = 9:1). The purified product was concentrated under reduced pressure to thereby obtain 4.23 g of pale-yellow oily 4-methyl-2-nitro-1-propoxybenzene (yield: 83%). ¹H-NMR (CDCl₃) δppm: 1.05 (3H, t, J = 7.4 Hz), 1.80-1.86 (2H, m), 2.33 (3H, s), 4.02 (2H, t, J = 6.4 Hz), 6.95 (1H, d, J = 8.5 Hz), 7.29 (1H d, J = 8.5 Hz), 7.62 (1H, s).

### Reference Example 2

### 5-Methyl-2-propoxyaniline

4-Methyl-2 -nitro-1-propoxybenzene (2.0 g, 10.2 mmol) and 5% palladium carbon (700 mg) were added to ethanol (30 ml), followed by conduction of catalytic reduction at room temperature under ordinary pressure. The catalyst was removed by Celite filtration, and the filtrate was concentrated under reduced pressure. The residue was dissolved in dichloromethane and dried over anhydrous magnesium sulfate. The resultant dry substance was concentrated under reduced pressure to thereby obtain 1.49 g of reddish-brown oily 5-methyl-2-propoxyaniline (yield: 89%). ¹H-NMR (CDCl₃ ) δppm: 1.05 (3H, t, J = 7.4 Hz), 1.76-1.86 (2H, m), 2.21 (3H, s), 3.73 (2H, brs), 3.91 (2H, t, J = 6.5 Hz), 6.49-6.50 (1H, m), 6.54 (1H, s), 6.66 (1H, d, J = 8. Hz).

### Reference Example 3

### Ethyl α-(hydroxymethylene)-4-methoxyphenyl acetate

Sodium hydride (60% in oil) (467 mg, 11.7 mmol) was added to a benzene solution (10 ml) of ethyl 4-methoxyphenyl acetate (2.0 g, 10.3 mmol), while being cooled with ice. The mixture was stirred at room temperature for 5 minutes. The stirred mixture was cooled with ice again; ethyl formate (1.02 ml, 12.6 mmol) was added thereto and stirred at room temperature for 3 hours. While being cooled with ice, water and ethyl acetate were added to the reaction mixture, and then 2N hydrochloric acid (6 ml) was added to separate the reaction mixture into two layers. The organic layer was concentrated under reduced pressure, and the residue was purified by silica gel column chromatography (n-hexane:ethyl acetate = 4:1). The purified product was concentrated under reduced pressure to thereby obtain 1.97 g of slightly reddish-brown oily ethyl α-(hydroxymethylene)-4-methoxyphenyl acetate (yield: 86%). The resulting object was purged with nitrogen and stored in a freezer.
¹H-NMR (CDCl₃) δppm: 1.28 (3H, t, J = 7.1 Hz), 3.81 (3H, s), 4.28 (2H, q, J = 7.1 Hz), 6.87 (2H, d, J = 8.8 Hz), 7.16-7.26 (3H, m), 12.02 (1H, d, J = 12.5 Hz).

### Example 1

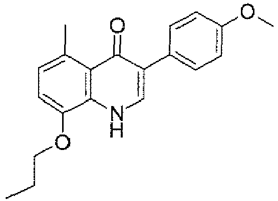

### 3-(4-Methoxyphenyl)-5-methyl-8-propoxy-1H-quinolin-4-one

270 mg of Amberlyst 15 (produced by Sigma-Aldrich Corporation) was added to a benzene solution (50 ml) of 5-methyl-2-propoxyaniline (1.49 g, 9.0 mmol) and ethyl α-(hydroxymethylene)-4-methoxyphenyl acetate (2.00 g, 9.0 mmol). The resulting mixture was heated under reflux for 6 hours using a Dean-Stark trap. The reaction mixture was then cooled to room temperature and filtered to remove resin. The filtrate was concentrated under reduced pressure. Diphenyl ether (2.5 ml) was added to the residue, and the mixture was then heated with a mantle heater and stirred for 50 minutes under reflux. The reaction mixture was cooled to room temperature, and then directly purified using silica gel column chromatography (dichloromethane:methanol = 80:1 → 60:1). The purified product was concentrated under reduced pressure to recrystallize the residue from ethyl acetate, thereby giving 600 mg of pale-yellow scaly crystal 3-(4-methoxyphenyl)-5-methyl-8-propoxy-1H-quinolin-4-one (yield: 21%).
Melting point: 192°C-193°C

Using appropriate starting materials, Examples 2 to 109 were prepared in the same manner as in Example 1.

### Example 2 (Reference Compound)

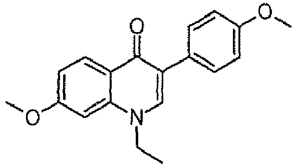

### 1-Ethyl-7-methoxy-3-(4-methoxyphenyl)-1H-quinolin-4-one White powder

Melting point: 129°C-131°C

### Example 3

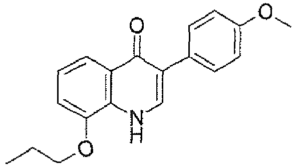

### 3-(4-Methoxyphenyl)-8-propoxy-1H-quinolin-4-one Yellow powder

Melting point: 231°C-233°C

### Example 4 (Reference Compound)

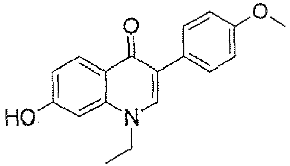

### 1-Ethyl-7-hydroxy-3-(4-methoxyphenyl)-1H-quinolin-4-one Pale-brown powder

¹H-NMR (DMSO-d₆) δppm: 1.35 (3H, t, J = 6.8 Hz), 3.76 (3H, s), 4.23 (2H, q, J = 6.9 Hz), 6.84-6.96 (4H, m), 7.64 (2H, d, J = 8.6 Hz), 8.09 (1H, s), 8.11 (1H, d, J = 8.8 Hz), 10.33 (1H, s).

### Example 5 (Reference Compound)

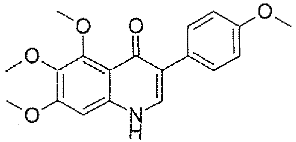

### 5,6,7-Trimethoxy-3-(4-methoxyphenyl)-1H-quinolin-4-one Pale-yellow powder

¹H-NMR (DMSO-d₆) δppm: 3.70 (3H, s), 3.76 (3H, s), 3.86 (3H, s), 3.93 (3H, s), 6.48 (1H, s), 6.95 (2H, d, J = 8.8 Hz), 7.59 (2H, d, J= 8.8 Hz), 8.22 (1H, s) 11.40 (1H, brs).

### Example 6

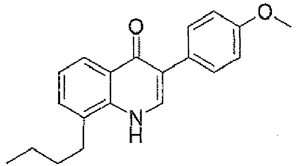

### 8-Butyl-3-(4-methoxyphenyl)-1H-quinolin-4-one

### Pale-brown powder

¹H-NMR (DMSO-d₆) δppm: 0.90 (3H, t, J = 7.2 Hz), 1.34-1.39 (2H, m), 1.55-1.59 (2H, m), 2.86 (2H, t, J=7.5Hz), 3.76 (3H, s), 6.95 (2H, d, J = 8.5 Hz), 7.25 (1H, t, J = 7.7 Hz), 7.46 (1H, d, J= 6.9 Hz), 7.62 (2H, d, J = 8.5 Hz), 7.92 (1H, s), 8.08 (1H, d, J = 8.0 Hz), 11.39 (1H, brs).

### Example 7

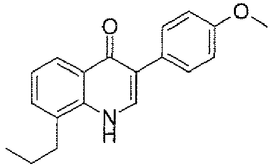

### 3-(4-Methoxyphenyl)-8-propyl-1H-quinolin-4-one

White powder

¹H-NMR (DMSO-d₆) δppm: 0.94 (3H, t, J = 7.2 Hz), 1.59-1.64 (2H, m), 2.83 (2H, t, J= 7.5 Hz), 3.75 (3H, s), 6.93-6.95 (2H, m), 7.25 (1H, t, J = 7.8Hz), 7.46 (1H, d, J = 6.0 Hz), 7.60-7.61 (2H, m), 7.92 (1H, s), 8.07-8.09 (1H, m), 11.40 (1H, brs).

### Example 8

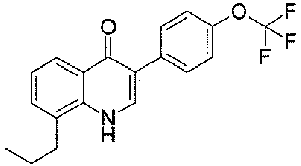

### 8-Propyl-3-(4-trifluoromethoxyphenyl)-1H-quinolin-4-one Pale-yellow powder

¹H-NMR (DMSO-d₆) δppm: 0.96 (3H, t, J = 7.2 Hz), 1.59-1.66 (2H, m), 2.85(2H, t, J = 7.6 Hz), 7.27 (1H, t, J = 7.9 Hz), 7.36 (2H, d, J = 8.7 Hz), 7.49 (1H, d, J = 7.0 Hz), 7.83 (2H, d, J = 8.7 Hz), 8.02 (1H, s), 8.09-8.10 (1H, m), 11.47 (1H, brs).

### Example 9

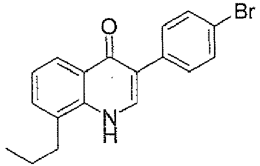

### 3-(4-Bromophenyl)-8-propyl-1H-quinolin-4-one

### White powder

¹H-NMR (DMSO-d₆) δppm: 0.95 (3H, t, J = 7.2 Hz), 1.58-1.65 (2H, m), 2.84 (2H, t, J = 7.6 Hz), 7.27 (1H, d, J = 7.9 Hz), 7.48 (1H, d, J = 7.1 Hz), 7.55 (2H, d, J = 8.5 Hz), 7.67 (2H, d, J = 8.5 Hz), 8.00 (1H, s), 8.08-8.09 (1H, m), 11.46 (1H, brs).

### Example 10

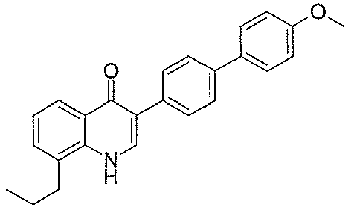

### 3-(4'-Methoxybiphenyl-4-yl)-8-propyl-1H-quinolin-4-one Pale-brown powder

¹H-NMR (DMSO-d₆) δppm: 0.95 (3H, t, J = 7.2 Hz), 1.59-1.66 (2H, m), 2.85 (2H, t, J = 7.6 Hz), 3.81 (3H, s), 7.00 (2H, d, J = 8.7 Hz), 7.28 (1H, t, J = 8.5 Hz), 7.48 (1H, d, J = 7.1 Hz), 7.60-7.64 (4H, m), 7.76 (2H, d, J = 8.2 Hz), 8.02 (1H, s), 8.11 (1H, d, J = 8.1 Hz), 11.45 (1H, brs).

### Example 11 (Reference Compound)

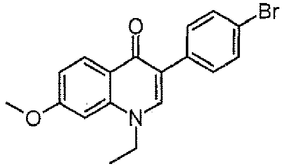

### 3-(4-Bromophenyl)-1-ethyl-7-methoxy-1H-quinolin-4-one White powder

¹H-NMR (DMSO-d₆) δppm: 1.37 (3H, t, J = 6.9 Hz), 3.91 (3H, s), 4.34 (2H, q, J = 7.0 Hz), 7.01-7.04 (2H, m), 7.54 (2H, d, J = 8.4 Hz), 7.69 (2H, d, J = 8.4 Hz), 8.20 (1H, d, J = 8.8 Hz), 8.24 (1H, s).

### Example 12 (Reference Compound)

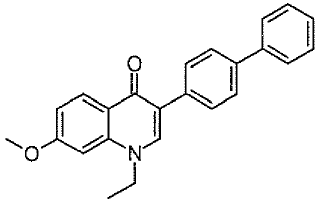

### 3-Biphenyl-4-yl-1-ethyl-7-methoxy-1H-quinolin-4-one White powder

¹H-NMR (DMSO-d₆)δppm: 1.38 (3H, t, J = 7.0 Hz), 3.91 (3H, s), 4.35 (2H, q, J = 7.0 Hz), 7.01-7.05 (2H, m), 7.34 (1H, t, J = 7.4 Hz), 7.45 (2H, t, J = 7.6 Hz), 7.65-7.68 (4H, m), 7.81 (2H, d, J = 8.3 Hz), 8.22-8.25 (2H, m).

### Example 13

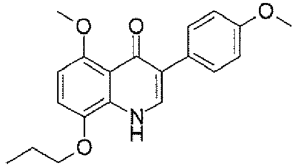

### 5-Methoxy-3-(4-methoxyphenyl)-8-propoxy-1H-quinolin-4-one Pale-brown powder

Melting point: 223°C-224°C

### Example 14

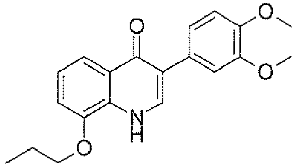

### 3-(3,4-Dimethoxyphenyl)-8-propoxy-1H-quinolin-4-one Pale-yellow powder

Melting point: 210°C-211°C

### Example 15

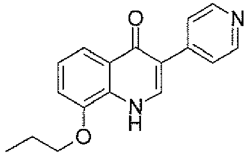

### 8-Propoxy-3-pylidine-4-yl-1H-quinolin-4-one Pale-brown powder

Melting point: 259°C-260°C

### Example 16

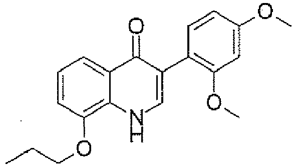

### 3-(2,4-Dimethoxyphenyl)-8-propoxy-1H-quinolin-4-one Pale-brown powder

Melting point: 231°C-232°C

### Example 17

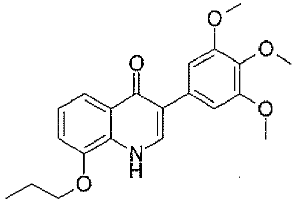

### 8-Propoxy-3-(3,4,5-trimethoxyphenyl)-1H-quinolin-4-one Pale-brown amorphous product

¹H-NMR (DMSO-d₆) δppm: 1.04 (3H, t, J = 7.3 Hz), 1.78-1.90 (2H, m), 3.67 (3H, s), 3.84 (6H, s), 4.12 (2H, t, J = 6.4 Hz), 7.00 (2H, s), 7.17-7.26 (2H, m), 7.74 (1H, d, J = 6.7 Hz), 7.99 (1H, d, J = 6.3 Hz), 11.47 (1H, d, J = 6.2 Hz).

### Example 18

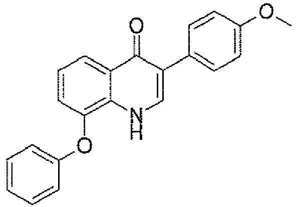

### 3-(4-Methoxyphenyl)-8-phenoxy-1H-quinolin-4-one

Pale-brown powder

Melting point: 250°C-251°C

### Example 19

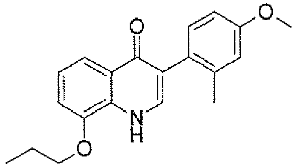

### 3-(4-Methoxy-2-methylphenyl)-8-propoxy-1H-quinolin-4-one Pale-yellow powder Melting point: 214°C-215°C

### Example 20

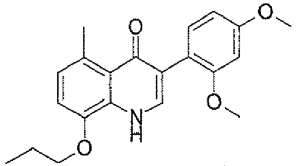

### 3-(2,4-Dimethoxyphenyl)-5-methyl-8-propoxy-1H-quinolin-4-one Pale-yellow powder

Melting point: 193°C-194°C

### Example 21

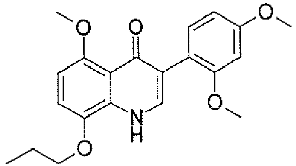

### 3-(2,4-Dimethoxyphenyl)-5-methoxy-8-propoxy-1H-quinolin-4-one Pale-gray powder

Melting point: 113°C-114°C

### Example 22

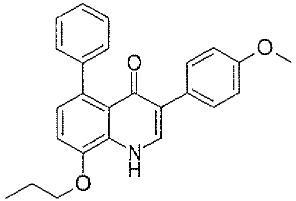

### 3-(4-Methoxyphenyl)-5-phenyl-8-propoxy-1H-quinolin-4-one Pale-brown powder

Melting point: 186°C-187°C

### Example 23

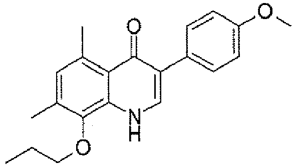

### 3-(4-Methoxyphenyl)-5,7-dimethyl-8-propoxy-1H-quinolin-4-one Pale-yellow powder

Melting point: 174°C-175°C

### Example 24

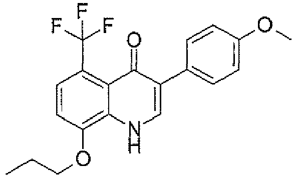

### 3-(4-Methoxyphenyl)-8-propoxy-5-trifluoromethyl-1H-quinolin-4-one Pale-yellow powder

Melting point: 220°C-221°C

### Example 25

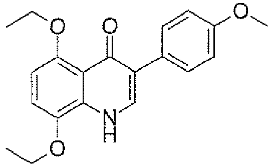

### 5,8-Diethoxy-3-(4-methoxyphenyl)-1H-quinolin-4-one Pale-yellow powder

Melting point: 182°C-183°C

### Example 26

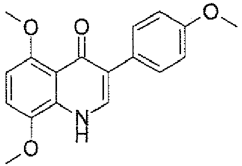

### 5,8-Dimethoxy-3-(4-methoxyphenyl)-1H-quinolin-4-one Pale-yellow powder

Melting point: 159°C-160°C

### Example 27

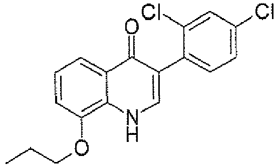

### 3-(2,4-Dichlorophenyl)-8-propoxy-1H-quinolin-4-one Green powder

Melting point: 189°C

### Example 28

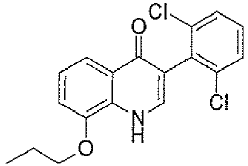

### 3-(2,6-Dichlorophenyl)-8-propoxy-1H-quinolin-4-one Pale-brown powder

Melting point: 193°C

### Example 29

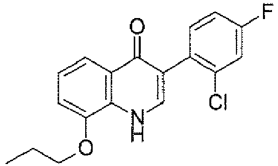

### 3-(2-Chloro-4-fluorophenyl)-8-propoxy-1H-quinolin-4-one Pale-orange powder

Melting point: 230°C

### Example 30

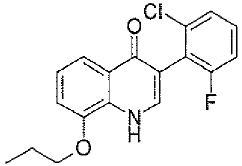

### 3-(2-Chloro-6-fluorophenyl)-8-propoxy-1H-quinolin-4-one Pale-brown powder

Melting point: 250°C

### Example 31

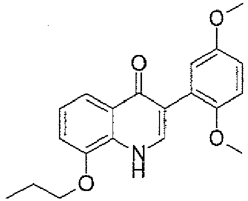

### 3-(2,5-Dimethoxyphenyl)-8-propoxy-1H-quinolin-4-one Pale-yellow powder

Melting point: 175°C

### Example 32

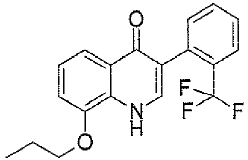

### 8-Propoxy-3-(2-trofluoromethylphenyl)-1H-quinolin-4-one White powder

Melting point: 224°C

### Example 33

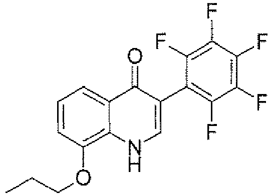

### 3-Pentafluorophenyl-8-propoxy-1H-quinolin-4-one Pale-brown powder

Melting point: 160°C

### Example 34

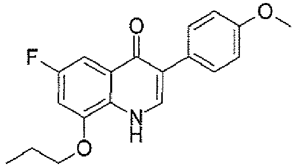

### 6-Fluoro-3-(4-methoxyphenyl)-8-propoxy-1H-quinolin-4-one Pale-yellow powder

Melting point: 153°C-154°C

### Example 35

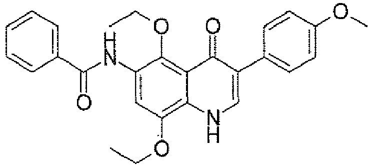

### N-[5,8-Diethoxy-3-(4-methoxyphenyl)-4-oxo-1,4-dihydroquinolin-6-yl]-benzamide

Pale-brown powder

Melting point: 120°C-121°C

### Example 36

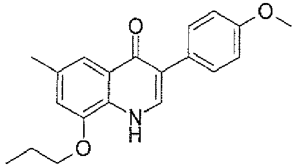

### 3-(4-Methoxyphenyl)-6-methyl-8-propoxy-1H-quinolin-4-one Pale-yellow powder

Melting point: 161°C-162°C

### Example 37

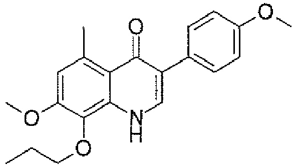

### 7-Methoxy-3-(4-methoxyphenyl)-5-methyl-8-propoxy-1H-quinolin-4-one

Pale-brown powder

Melting point: 195°C-196°C

### Example 38

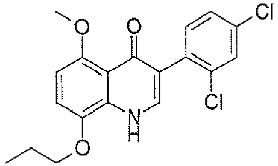

### 3-(2,4-Dichlorophenyl)-5-methoxy-8-propoxy-1H-quinolin-4-one

White powder

Melting point: 125°C

### Example 39

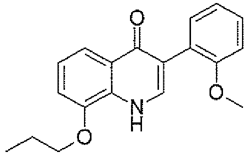

### 3-(2-Methoxyphenyl)-8-propoxy-1H-quinolin-4-one White powder

Melting point: 218°C-220°C

### Example 40

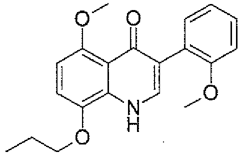

### 5-Methoxy-3-(2-methoxyphenyl)-8-propoxy-1H-quinolin-4-one White powder

Melting point: 239°C-241°C

### Example 41

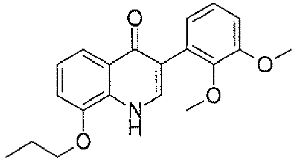

### 3-(2,3-Dimethoxyphenyl)-8-propoxy-1H-quinolin-4-one Pale-yellow powder

Melting point: 253°C-255°C

### Example 42

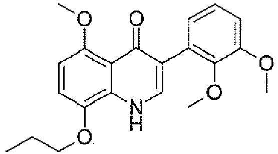

### 3-(2,3-Dimethoxyphenyl)-5-methoxy-8-propoxy-1H-quinolin-4-one Pale-yellow powder

Melting point: 145°C-148°C

### Example 43

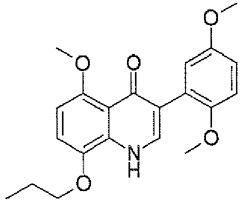

### 3-(2,5-Dimethoxyphenyl)-5-methoxy-8-propoxy-1H-quinolin-4-one Pale-yellow powder

Melting point: 179°C-180°C

### Example 44

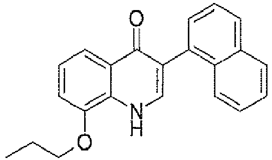

### 3-Naphthalen-1-yl-8-propoxy-1H-quinolin-4-one Pale-yellow powder

Melting point: 255°C-256°C

### Example 45

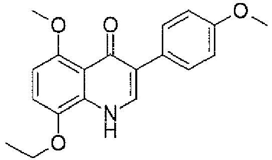

### 8-Ethoxy-5-methoxy-3-(4-methoxyphenyl)-1H-quinolin-4-one Pale-yellow powder

Melting point: 117°C-119°C

### Example 46

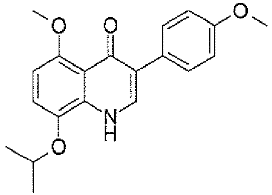

### 8-Isopropoxy-5-methoxy-3-(4-methoxyphenyl)-1H-quinolin-4-one Pale-yellow powder

Melting point: 213°C-214°C

### Example 47

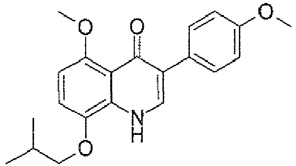

### 8-Isobutoxy-5-methoxy-3-(4-methoxyphenyl)-1H-quinolin-4-one Pale-yellow powder

Melting point: 242°C-244°C

### Example 48

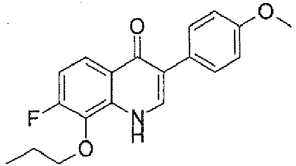

### 7-Fluoro-3-(4-methoxyphenyl)-8-propoxy-1H-quinolin-4-one Pale-yellow powder

Melting point: 160°C-161°C

### Example 49

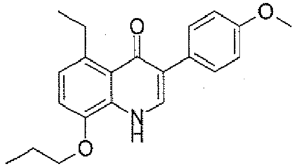

### 5-Ethyl-3-(4-methoxyphenyl)-8-propoxy-1H-quinolin-4-one Pale-yellow powder

Melting point: 169°C-170°C

### Example 50

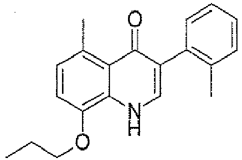

### 5-Methyl-8-propoxy-3-o-tolyl-1H-quinolin-4-one Pale-yellow powder

Melting point: 201°C-202°C

### Example 51

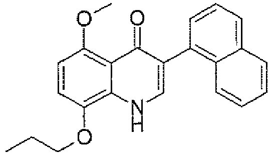

### 5-Methoxy-3-naphthalen-1-yl-8-propoxy-1H-quinolin-4-one Pale-yellow powder

Melting point: 130°C-133°C

### Example 52

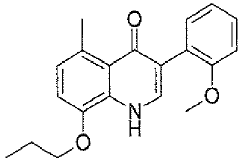

### 3-(2-Methoxyphenyl)-5-methyl-8-propoxy-1H-quinolin-4-one White powder

Melting point: 221°C-223°C

### Example 53

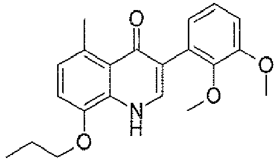

### 3-(2,3-Dimethoxyphenyl)-5-methyl-8-propoxy-1H-quinolin-4-one Pale-yellow powder

Melting point: 170°C-171°C

### Example 54

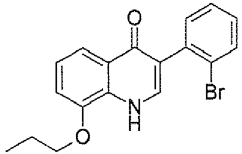

### 3-(2-Bromophenyl)-8-propoxy-1H-quinolin-4-one Pale-yellow powder

Melting point: 200°C-203°C

### Example 55

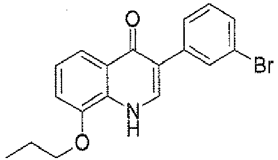

### 3-(3-Bromophenyl)-8-propoxy-1H-quinolin-4-one

Pale-yellow powder

Melting point: 107°C-108°C

### Example 56

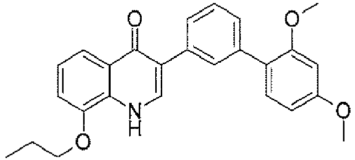

### 3-(2',4'-Dimethoxybiphenyl-3-yl)-8-propoxy-1H-quinolin-4-one Pale-yellow powder

Melting point: 81°C-84°C

### Example 57

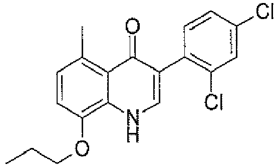

### 3-(2,4-Dichlorophenyl)-5-methyl-8-propoxy-1H-quinolin-4-one Pale-yellow powder

Melting point: 103°C-106°C

### Example 58

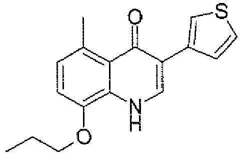

### 5-Methyl-8-propoxy-3-thiophen-3-yl-1H-quinolin-4-one Pale-brown powder

Melting point: 104°C-107°C

### Example 59

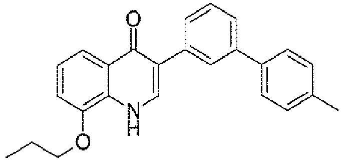

### 3-(4'-Methylbiphenyl-3-yl)-8-propoxy-1H-quinolin-4-one Pale-orange powder

Melting point: 189°C-193°C

### Example 60

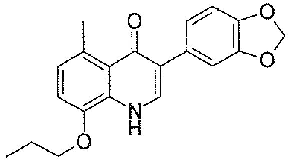

### 3-Benzo[1,3]dioxol-5-yl-5-methyl-8-propoxy-1H-quinolin-4-one Pale-brown powder

Melting point: 110°C-115°C

### Example 61

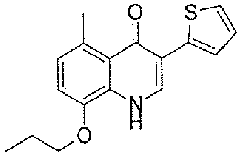

### 5-Methyl-8-propoxy-3-thiophen-2-yl-1H-quinolin-4-one Light-green powder

Melting point: 104°C-105°C

### Example 62

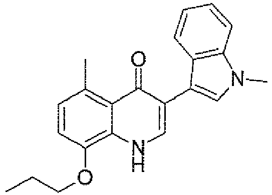

### 5-Methyl-3-(1-methyl-1H-indol-3-yl)-8-propoxy-1H-quinolin-4-one Pale-brown powder

Melting point: 106°C-109°C

### Example 63

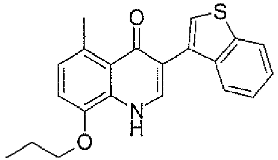

### 3-Benzo[b]thiophen-3-yl-5-methyl-8-propoxy-1H-quinolin-4-one Pale-brown powder

Melting point: 80°C-82°C

### Example 64

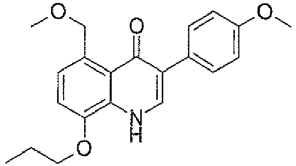

### 5-Methoxymethyl-3-(4-methoxyphenyl)-8-propoxy-1H-quinolin-4-one Pale-brown powder

Melting point: 81°C-83°C

### Example 65

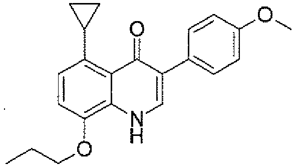

### 5-Cyclopropyl-3-(4-methoxyphenyl)-8-propoxy-1H-quinolin-4-one White powder

Melting point: 168°C-170°C

### Example 66

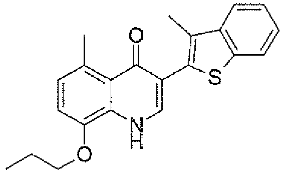

### 5-Methyl-3-(3-methylbenzo[b]thiophen-2-yl)-8-propoxy-1H-quinolin-4-one

Pale-yellow powder

Melting point: 90°C-92°C

### Example 67

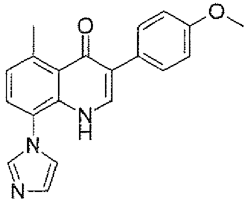

### 8-Imidazol-1-yl-3-(4-methoxyphenyl)-5-methyl-1H-quinolin-4-one Pale-yellow powder

Melting point: 196°C-198°C

### Example 68

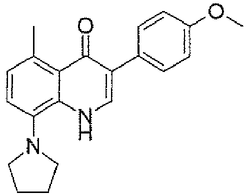

### 3-(4-Methoxyphenyl)-5-methyl-8-pyrrolidin-1-yl-1H-quinolin-4-one Pale-yellow powder

Melting point: 177°C-178°C

### Example 69

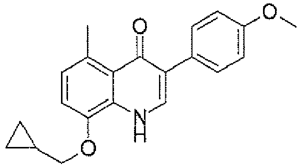

### 8-Cyclopropylmethoxy-3-(4-methoxyphenyl)-5-methyl-1H-quinolin-4-one

White powder
Melting point: 182°C-183°C

### Example 70

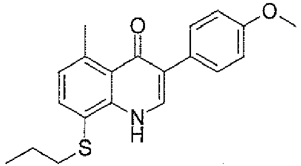

### 3-(4-Methoxyphenyl)-5-methyl-8-propylsulfanyl-1H-quinolin-4-one Pale-yellow powder

Melting point: 132°C-135°C

### Example 71

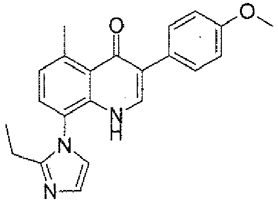

### 8-(2-Ethylimidazol-1-yl)-3-(4-methoxyphenyl)-5-methyl-1H-quinolin-4-one

Pale-yellow powder

Melting point: 258°C-260°C

### Example 72

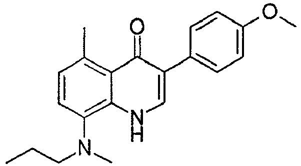

### 3-(4-Methoxyphenyl)-5-methyl-8-(methyl-propyl-amino)-1H-quinolin-4-one

White powder

Melting point: 159°C-161°C

### Example 73

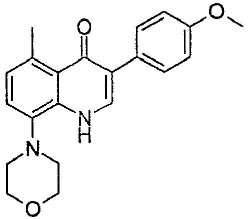

### 3-(4-Methoxyphenyl)-5-methyl-8-morpholin-4-yl-1H-quinolin-4-one Pale-brown powder

Melting point: 260°C-263°C

### Example 74 (Reference Compound)

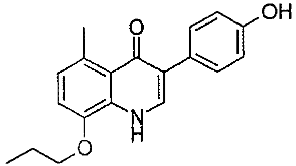

### 3-(4-Hydroxyphenyl)-5-methyl-8-propoxy-1H-quinolin-4-one White powder

Melting point: 265°C-267°C

### Example 75 (Reference Compound)

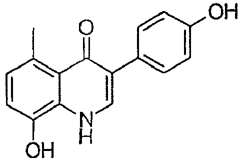

### 8-Hydroxy-3-(4-hydroxyphenyl)-5-methyl-1H-quinolin-4-one Pale-yellow powder

Melting point: 270°C-275°C (decomposition)

### Example 76

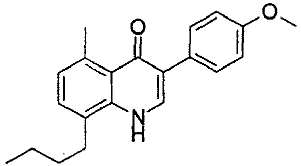

### 8-Butyl-3-(4-methoxyphenyl)-5-methyl-1H-quinolin-4-one Pale-yellow powder

Melting point: 186°C-188°C

### Example 77

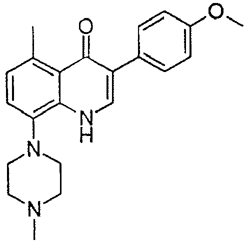

### 3-(4-Methoxyphenyl)-5-methyl-8-(4-methylpiperazin-1-yl)-1H-quinolin-4-one Pale-brown powder

Melting point: 214°C-215°C

### Example 78

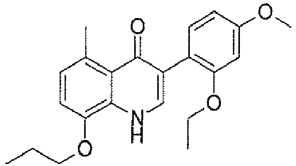

### 3-(2-Ethoxy-4-methoxyphenyl)-5-methyl-8-propoxy-1H-quinolin-4-one White powder

Melting point: 191°C-192°C

### Example 79

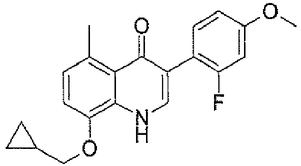

### 8-Cyclopropylmethoxy-3-(2-fluoro-4-methoxyphenyl)-5-methyl-1H-quinolin-4-one

White powder

Melting point: 198°C-199°C

### Example 80

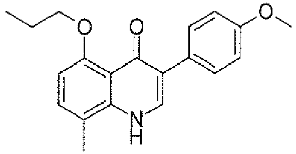

### 3-(4-Methoxyphenyl)-8-methyl-5-propoxy-1H-quinolin-4-one Pale-yellow powder

Melting point: 156°C-158°C

### Example 81

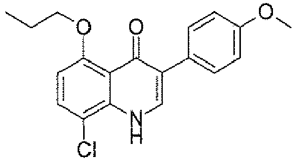

### 8-Chloro-3-(4-methoxyphenyl)-5-propoxy-1H-quinolin-4-one Pale-yellow powder

Melting point: 145°C-147°C

### Example 82

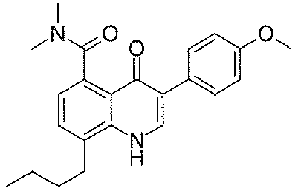

### 8-Butyl-3-(4-methoxyphenyl)-4-oxo-1,4-dihydroquinolin-5-carboxylic acid dimethylamide

Pale-yellow powder

Melting point: 198°C-200°C

### Example 83

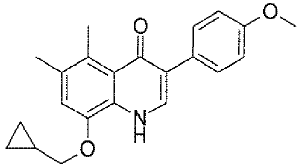

### 8-Cyclopropylmethoxy-3-(4-methoxyphenyl)-5,6-dimethyl-1H-quinolin-4-one

Pale-brown powder

Melting point: 99°C-101°C

### Example 84

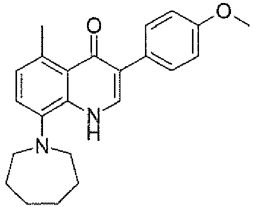

### 8-Azepan-1-yl-3-(4-methoxyphenyl)-5-methyl-1H-quinolin-4-one Yellow powder

Melting point: 249°C-250°C

### Example 85

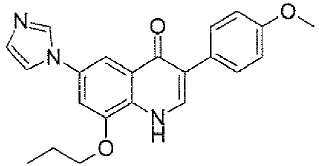

### 6-Imidazol-1-yl-3-(4-methoxyphenyl)-8-propoxy-1H-quinolin-4-one

Pale-brown powder

Melting point: 236°C-237°C

### Example 86

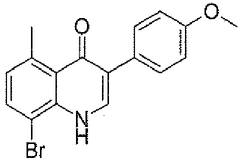

### 8-Bromo-3-(4-methoxyphenyl)-5-methyl-1H-quinolin-4-one Pale-yellow powder

Melting point: 185°C-186°C

### Example 87

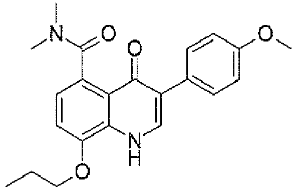

### 3-(4-Methoxyphenyl)-4-oxo-8-propoxy-1,4-dihydroquinolin-5-carboxylic acid dimethylamide

Pale-gray powder

Melting point: 218°C-220°C

### Example 88

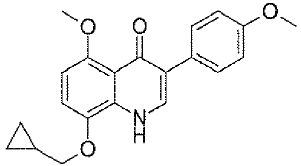

### 8-Cyclopropylmethoxy-5-methoxy-3-(4-methoxyphenyl)-1H-quinolin-4-one

Pale-brown powder

Melting point: 212°C-2144°C

### Example 89

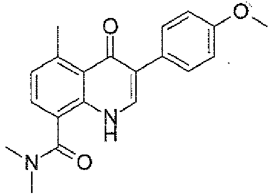

### 3-(4-Methoxyphenyl)-5-methyl-4-oxo-1,4-dihydroquinolin-8-carboxylic acid dimethylamide

Yellow powder

Melting point: 158°C-160°C

### Example 90

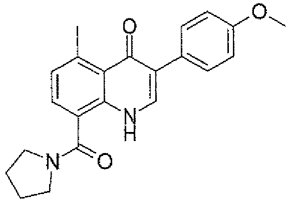

### 3-(4-Methoxyphenyl)-5-methyl-8-(pyrrolidine-1-carbonyl)-1H-quinolin-4-one

Pale-brown powder

Melting point: 193°C-195°C

### Example 91

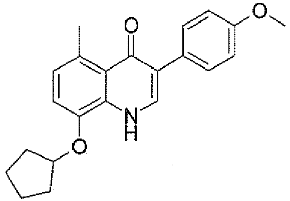

### 8-Cyclopentyloxy-3-(4-methoxyphenyl)-5-methyl-1H-quinolin-4-one Pale-yellow powder

Melting point: 237°C-239°C

### Example 92

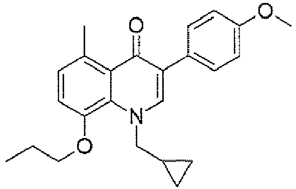

### 1-Cyclopropylmethyl-3-(4-methoxyphenyl)-5-methyl-8-propoxy-1H-quinolin-4-one

White powder

Melting point: 100°C-101°C

### Example 93

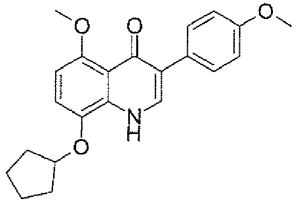

### 8-Cyclopentyloxy-5-methoxy-3-(4-methoxyphenyl)-1H-quinolin-4-one Yellow green powder

Melting point: 213°C-215°C

### Example 94

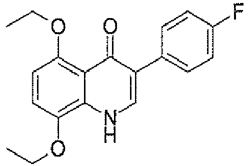

### 5,8-Diethoxy-3-(4-fluorophenyl)-1H-quinolin-4-one Pale-yellow powder

Melting point: 232°C-234°C

### Example 95

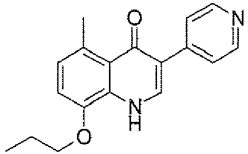

### 5-Methyl-8-propoxy-3-pyridin-4-yl-1H-quinolin-4-one Pale-orange powder

Melting point: 112°C-113°C

### Example 96

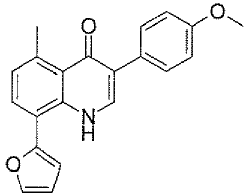

### 8-Furan-2-yl-3-(4-methoxyphenyl)-5-methyl-1H-quinolin-4-one Pale-yellow powder

Melting point: 129°C-131°C

### Example 97

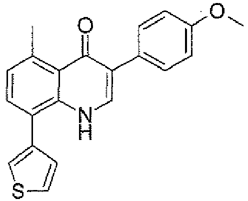

### 3-(4-Methoxyphenyl)-5-methyl-8-thiophen-3-yl-1H-quinolin-4-one White powder

Melting point: 189°C-190°C

### Example 98

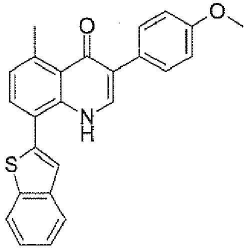

### 8-Benzo[b]thiophen-2-yl-3-(4-methoxyphenyl)-5-methyl-1H-quinolin-4-one

White powder

Melting point: 229°C-231°C

### Example 99

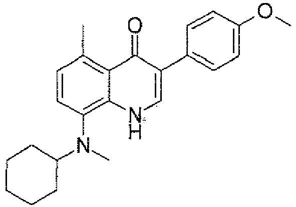

### 8-(N-Cyclohexyl-N-methylamino)-3-(4-methoxyphenyl)-5-methyl-1H-quinolin-4-one White powder

Melting point: 186°C-187°C

### Example 100

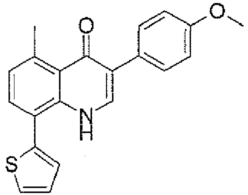

### 3-(4-Methoxyphenyl)-5-methyl-8-thiophen-2-yl-1H-quinolin4-one Orange powder

Melting point: 197°C-199°C

### Example 101

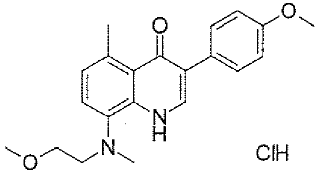

### 8-[(2-Methoxyethyl)methyl-amino]-3-(4-methoxyphenyl)-5-methyl-1H-quinolin-4-one hydrochloride

Pale-yellow powder

Melting point: 90°C-93°C

### Example 102

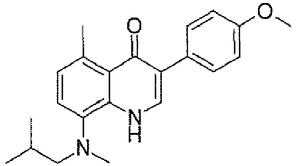

### 8-(N-Isobutyl-N-methyl-amino)-3-(4-methoxyphenyl)-5-methyl-1H-quinolin-4-one

White powder

Melting point: 111°C-113°C

### Example 103

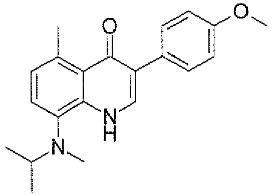

### 8-(N-Isopropyl-N-methylamino)-3-(4-methoxyphenyl)-5-methyl-1H-quinolin-4-one

White powder

Melting point: 186°C-187°C

### Example 104

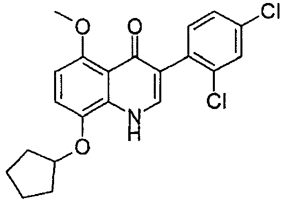

### 8-Cyclopentyloxy-3-(2,4-dichlorophenyl)-5-methoxy-1H-quinolin-4-one

White powder

Melting point: 266°C-268°C

### Example 105

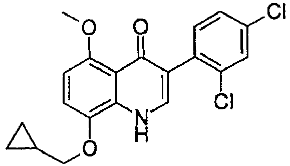

### 8-Cyclopropylmethoxy-3-(2,4-dichlorophenyl)-5-methoxy-1H-quinolin-4-one

Pale-brown powder

Melting point: 254°C-256°C

### Example 106 (Reference Compound)

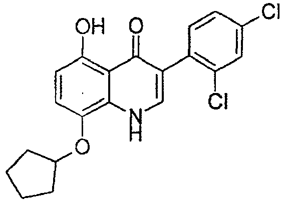

### 8-Cyclopentyloxy-3-(2,4-dichlorophenyl)-5-hydroxy-1H-quinolin-4-one

Yellow powder

Melting point: 154°C-155°C

### Example 107 (Reference Compound)

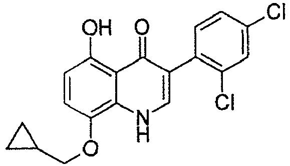

### 8-Cyclopropylmethoxy-3-(2,4-dichlorophenyl)-5-hydroxy-1H-quinolin-4-one

Yellow powder

Melting point: 163°C-165°C

### Example 108

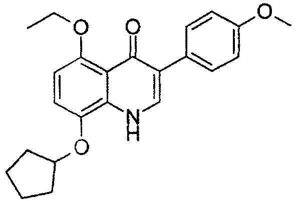

### 8-Cyclopentyloxy-5-ethoxy-3-(4-methoxyphenyl)-1H-quinolin-4-one

Pale-yellow powder

Melting point: 204°C-206°C

### Example 109

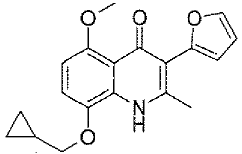

### 8-Cyclopropylmethoxy-3-furan-2-yl-5-methoxy-2-methyl-1H-quinolin-4-one

Pale-yellow powder

Melting point: 189°C-190°C

### Pharmacological Test 1

### Evaluation of improvement of mitochondrial function using human neuroblastoma cell lines SH-SY5Y treated with 1-methyl-4-phenylpyridinium (MPP⁺)

In human neuroblastoma cell lines SH-SY5Y in which mitochondrial function was damaged by MPP⁺ treatment (Bollimuntha S. et al., J Biol Chem, 280, 2132-2140 (2005) and Shang T. et al., J Biol Chem, 280, 34644-34653 (2005)), the improvement of mitochondrial function was evaluated on the basis of the measurement value for mitochondrial oxidation reduction activity using Alamar Blue fluorescent dye after the compound addition (Nakai M. et al, Exp Neurol, 179, 103-110 (2003)).

The human neuroblastoma cell lines SH-SY5Y were cultured in Dulbecco's Modified Eagle's Medium containing 10% fetal bovine serum (DMEM containing 50 units/ml penicillin and 50 µg/ml streptomycin as antibiotics) at 37°C in the presence of 5% carbon dioxide. Cells were scattered on a poly-D-lysine-coated 96-well black plate at a concentration of 3-6 x 10⁴ cells/cm²
(medium amount: 100 µl/well) and cultured in the medium for two days. Further, the medium was changed to DMEM containing a 1% N2 supplement (N2-DMEM) or to a medium (100 µl/well) in which 1.5 mM MPP⁺ was dissolved. The cells were cultured therein for 39 to 48 hours, and then subjected to a mitochondrial oxidation reduction activity measurement system. A sample compound that had been previously dissolved in dimethyl sulfoxide (DMSO) was diluted with N2-DMEM and added in a volume of 10 µl/well 24 hours before the activity measurement (final compound concentration: 0.01 to 1 µg/ml).

After removal of the medium by suction, a balanced salt solution containing 10% Alamar Blue (154 mM sodium chloride, 5.6 mM potassium chloride, 2.3 mM calcium chloride, 1.0 mM magnesium chloride, 3.6 mM sodium hydrogen carbonate, 5 mM glucose, 5 mM HEPES, pH 7.2) was added in a volume of 100 µl/well, and reacted in an incubator at 37°C for 1 hour. The fluorescent intensity was detected using a fluorescence detector (a product of Hamamatsu Photonics K.K., excitation wavelength: 530 nm, measurement wavelength: 580 nm) to thereby measure the mitochondrial oxidation reduction activity.

The fluorescent intensity of the well of the cells cultured in a medium containing MPP⁺ and each of the sample compounds was relatively evaluated based on the 100% fluorescent intensity of the well of the cells cultured in a medium containing DMSO alone (final concentration: 0.1%). When the MPP⁺-induced cell group exhibited higher florescent intensity than the cell group cultured in DMSO alone, the test compound was judged to have improved the mitochondrial function.

**Table 1**

| Improvement of mitochondrial function using human neuroblastoma cell lines SH-SY5Y treated with 1-methyl-4-phenylpyridinium (MPP⁺) | | | | | | | |
|---|---|---|---|---|---|---|---|
| Test Compound | Fluorescence Intensity (%) | | | | | | |
| Concentration (µg/ml) | | 0 | 0.01 | 0.03 | 0.1 | 0.3 | 1 |
| Compound of Example | 1 | 51 | 58 | 68 | 73 | 66 | 46 |
| Compound of Example | 2 | 54 | 71 | 73 | 74 | 77 | 81 |
| Compound of Example | 3 | 49 | 68 | 77 | 77 | 83 | 67 |
| Compound of Example | 13 | 48 | 56 | 65 | 74 | 69 | 62 |
| Compound of Example | 18 | 28 | 29 | 45 | 44 | 47 | 30 |
| Compound of Example | 20 | 52 | 65 | 67 | 74 | 78 | 77 |
| Compound of Example | 21 | 54 | 65 | 68 | 77 | 82 | 84 |
| Compound of Example | 23 | 44 | 51 | 63 | 67 | 68 | 59 |
| Compound of Example | 24 | 42 | 50 | 57 | 64 | 63 | 36 |
| Compound of Example | 32 | 45 | 49 | 53 | 54 | 57 | 61 |
| Compound of Example | 34 | 42 | 47 | 53 | 53 | 63 | 53 |
| Compound of Example | 37 | 43 | 47 | 54 | 55 | 60 | 59 |
| Compound of Example | 38 | 39 | 47 | 54 | 67 | 75 | 65 |
| Compound of Example | 51 | 34 | 37 | 45 | 54 | 65 | 49 |
| Compound of Example | 58 | 39 | 42 | 53 | 60 | 61 | 55 |
| Compound of Example | 60 | 48 | 52 | 65 | 75 | 70 | 44 |
| Compound of Example | 63 | 33 | 40 | 48 | 57 | 43 | 21 |
| Compound of Example | 64 | 44 | 51 | 56 | 63 | 53 | 38 |
| Compound of Example | 65 | 45 | 59 | 73 | 78 | 66 | 20 |
| Compound of Example | 67 | 40 | 45 | 54 | 61 | 57 | 42 |
| Compound of Example | 68 | 42 | 49 | 56 | 62 | 57 | 33 |
| Compound of Example | 70 | 42 | 48 | 56 | 61 | 50 | 22 |
| Compound of Example | 82 | 53 | 63 | 62 | 62 | 69 | 81 |
| Compound of Example | 83 | 54 | 65 | 70 | 67 | 62 | 29 |
| Compound of Example | 86 | 53 | 60 | 65 | 65 | 65 | 52 |
| Compound of Example | 87 | 55 | 57 | 57 | 60 | 65 | 66 |
| Compound of Example | 95 | 46 | 51 | 56 | 57 | 61 | 47 |
| Compound of Example | 96 | 51 | 54 | 64 | 68 | 60 | 23 |
| Compound of Example | 109 | 47 | 49 | 62 | 65 | 82 | 73 |

### Pharmacological Test 2

### Evaluation of dopaminergic neuronal protective activity using C57BL/6 mouse treated with 1-methyl-4-phenyl 1,2,3,6-tetrahydro pyridine (MPTP)

Using a mouse having MPTP-induced dopaminergic neurons (Chan P. et al., J Neurochem, 57, 348-351 (1991)), the dopaminergic neuronal protective activity was evaluated based on the protein levels of tyrosine hydroxylase (TH) and dopamine transporter (DAT), which are dopaminergic neuronal marker proteins, and a dopamine content in the brain corpus striatum region after the compound administration (Mori A. et al., Neurosci Res, 51, 265-274(2005)).

A male C57BL/6 mouse (provided by Japan Charles River Inc., 10 to 12 weeks) was used as a test animal. MPTP was dissolved in a physiological salt solution so that the concentration was 4 mg/ml, and then administered to the mouse subcutaneously in a volume of 10 ml/kg. A test compound was suspended in a 5% gum arabic/physiological salt solution (w/v) so that the concentration was 1 mg/ml. Each of the test compounds or solvents thereof was orally administered to the mouse after 30 minutes, 24 hours, and 48 hours of the MPTP administration. The mouse was decapitated after 72 hours of the MPTP administration, the brain was removed, and each side of the striatum was dissected.

The left striatum was used as a sample to detect the protein level by Western blot analysis. Each tissue was homogenized in a HEPES buffer sucrose solution (0.32 M sucrose, 4 µg/ml pepstatin, 5 µg/ml aprotinin, 20 µg/ml trypsin inhibitor, 4 µg/ml leupeptin, 0.2 mM phenylmethanesulfonyl fluoride, 2 mM ethylenediaminetetraacetic acid (EDTA), 2 mM ethylene glycol bis (β aminoethyl ether) tetraacetic acid, 20 mM HEPES, pH 7.2), and assayed for protein using a bicinchoninic acid kit for protein assay (provided by Pierce Corporation). Each homogenized sample, having an equal amount of protein that had been dissolved in a Laemmli sample buffer solution, was subjected to electrophoresis through a sodium dodecyl sulfate polyacrylamide gel. The protein separated by electrophoresis was electrically transferred to polyvinylidene fluoride membrane. The membrane was reacted with a specific primary antibody for TH, DAT, and housekeeping proteins, i.e., α1 subunit of Na⁺/K⁺-ATPase and actin (Na⁺/K⁺-ATPase is a product of UpState Biotechnology Inc.; others are products of Chemi-Con Corporation). Subsequently, a horseradish peroxidase-labeled secondary antibody (a product of Amersham K.K.) for each primary antibody was fixed, and the chemiluminescence associated with enzyme activity of peroxidase was detected using a X-ray film. The density of the protein band on the film was analyzed using a densitometer (a product of Bio-rad Laboratories Inc.) to obtain the TH value relative to Na⁺/K⁺-ATPase and the DAT value relative to actin.

The right striatum, the tissue weight of which was measured immediately after dissection, was used as an analysis sample for determining the dopamine content. Each tissue was homogenized in a 0.1 N perchloric acid solution containing isoproterenol as an internal standard substance of the measurement, using an ultrasonic homogenizer while being cooled with ice. The supernatant obtained from 20,000 g of homogenate that had been centrifuged at 4°C for 15 minutes was subjected to a high-performance liquid chromatography with a reversed phase column (a product of Eicom Corporation). A mobile phase 15% methanol 0.1 M citric acid/0.1 M sodium acetate buffer solution (containing 190 mg/L 1-sodium octane sulfonate and 5 mg/L EDTA, pH 3.5) was flowed at a rate of 0.5 ml/min, and the dopamine peak of each sample was detected using an electrochemical detector (applied voltage: +750 mV vs. Ag/AgCl, a product of Eicom Corporation). With reference to the identified dopamine peak, the dopamine content per tissue weight was calculated in each sample using analysis software (a product of Gilson Inc.).

In both analyses, the value of the sample derived from the MPTP-induced mouse in which only the test compound or the solvent was administered was expressed relative to the value of the sample derived from the mouse without MPTP treatment (100%). Values were analyzed statistically using a nonclinical statistical analysis system, and values of significance probability less than 0.05 were defined as significant. In the MPTP-induced mouse, when the test drug group showed an increase in protein level compared to the solvent group, and a significant difference was observed between these groups in the t-assay, the test drug was judged to have dopamine neuroprotective activity.

**Table 2**

| Protein level of tyrosine hydroxylase (TH) in the brain corpus striatum region (% of control) | | |
|---|---|---|
| Test Compound | (% of Control) | |
| Dosage | 0 mg/kg | 10 mg/kg |
| Compound of Example 1 | 51.6 | 86.2 |
| Compound of Example 65 | 50.2 | 65.2 |
| Compound of Example 67 | 57.7 | 77.9 |
| Compound of Example 70 | 49.5 | 90.2 |

**Table 3**

| Protein level of dopamine transporter (DAT) in the brain corpus striatum region (% of control) | | |
|---|---|---|
| Test Compound | (% of Control) | |
| Dosage | 0 mg/kg | 10 mg/kg |
| Compound of Example 1 | 29.5 | 84.7 |
| Compound of Example 65 | 43.1 | 73.0 |
| Compound of Example 67 | 38.4 | 50.9 |
| Compound of Example 70 | 39.6 | 64.1 |

**Table 4**

| Dopamine content in the brain corpus striatum region (% of control) | | |
|---|---|---|
| Test Compound | (% of Control) | |
| Dosage | 0 mg/kg | 10 mg/kg |
| Compound of Example 1 | 4.8 | 39.6 |
| Compound of Example 65 | 4.0 | 31.2 |
| Compound of Example 67 | 12.0 | 26.7 |
| Compound of Example 70 | 8.9 | 26.5 |

## Claims

1. Aquinolone compound represented by the following Formula (1) for use in the therapy and/or prophylaxis of neurodegenerative diseases, diseases induced by neurological dysfunction, or diseases induced by deterioration of mitochondrial function: 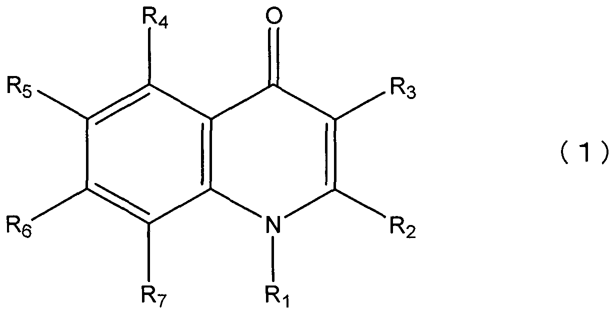 or a salt thereof, wherein:
R₁ represents hydrogen;
R₂ represents hydrogen or C₁-C₆ alkyl; and
R₃ represents phenyl, naphthyl, pyridyl, furyl, thienyl, indolyl, benzodioxolyl or benzothienyl, wherein the aromatic or heterocyclic ring represented by R₃ may be substituted with one or more substituents selected from the group consisting of the following substituents (1) to (6):
(1) C₁-C₆ alkyl,
(2) halogen-substituted C₁-C₆ alkyl,
(3) C₁-C₆ alkoxy,
(4) halogen-substituted C₁-C₆ alkoxy,
(5) phenyl optionally having one or more substituents selected from the group consisting of C₁-C₆ alkyl and C₁-C₆ alkoxy, and
(6) halogen;
R₄ represents hydrogen, C₁-C₆ alkyl, halogen-substituted C₁-C₆ alkyl, C₁-C₆ alkoxy,
C₁-C₆ alkoxy C₁-C₆ alkyl, phenyl, cyclo C₃-C₈ alkyl, or carbamoyl optionally having one or two C₁-C₆ alkyl groups;
R₅ represents hydrogen, C₁-C₆ alkyl, halogen, C₁-C₆ alkoxy, benzoylamino, or imidazolyl,
R₆ represents hydrogen, halogen, C₁-C₆ alkyl, or C₁-C₆ alkoxy; and
R₇ represents any of the following groups (1) to (17) :
(1) C₁-C₆ alkyl,
(2) C₁-C₆ alkoxy,
(3) phenoxy,
(4) cyclo C₃-C₈ alkyloxy,
(5) halogen,
(6) C₁-C₆ alkylthio,
(7) amino optionally having one or two substituents selected from the group consisting of C₁-C₆ alkyl, C₁-C₆ alkoxy C₁-C₆ alkyl, and cyclo C₃-C₈ alkyl,
(8) carbamoyl optionally having one or two C₁-C₆ alkyl groups,
(9) pyrrolidinyl,
(10) azepanyl,
(11) morpholinyl,
(12) piperazinyl optionally having one or two C₁-C₆ alkyl groups,
(13) imidazolyl optionally having one or two C₁-C₆ alkyl groups,
(14) furyl,
(15) thienyl,
(16) benzothienyl, and
(17) pyrrolidinylcarbonyl.

2. The quinolone compound for use according to claim 1, wherein the neurodegenerative disease is selected from the group consisting of Parkinson's disease, Parkinson's syndrome, juvenile parkinsonism, striatonigral degeneration, progressive supranuclear palsy, pure akinesia, Alzheimer's disease, Pick's disease, prion disease, corticobasal degeneration, diffuse Lewy body disease, Huntington's disease, chorea-acanthocytosis, benign hereditary chorea, paroxysmal choreoathetosis, essential tremor, essential myoclonus, Gilles de la Tourette's syndrome, Rett's syndrome, degenerative ballism, dystonia musculorum deformans, athetosis, spasmodic torticollis, Meige syndrome, cerebral palsy, Wilson's disease, Segawa's disease, Hallervorden-Spatz syndrome, neuroaxonal dystrophy, pallidal atrophy, spinocerebellar degeneration, cerebral cortical atrophy, Holmes-type cerebellar atrophy, olivopontocerebellar atrophy, hereditary olivopontocerebellar atrophy, Joseph disease, dentatorubropallidoluysian atrophy, Gerstmann-Straussler-Scheinker disease, Friedreich's ataxia, Roussy-Levy syndrome, May-White syndrome, congenital cerebellar ataxia, hereditary episodic ataxia, ataxia telangiectasia, amyotrophic lateral sclerosis, progressive bulbar palsy, spinal progressive muscular atrophy, spinobulbar muscular atrophy, Werdnig-Hoffmann disease, Kugelberg-Welander disease, hereditary spastic paraparesis, syringomyelia, syringobulbia, Arnold-Chiari malformation, Stiffman syndrome, Klippel-Feil syndrome, Fazio-Londe syndrome, lower myelopathy, Dandy-Walker syndrome, spina bifida, Sjogren-Larsson syndrome, radiation myelopathy, age-related macular degeneration, and cerebral apoplexy selected from the group consisting of cerebral infarction and cerebral hemorrhage and/or associated dysfunction or neurologic deficits.

3. The quinolone compound for use according to claim 1, wherein the disease induced by neurological dysfunction is selected from the group consisting of spinal cord injury, chemotherapy-induced neuropathy, diabetic neuropathy, radiation damage, and a demyelinating disease selected from the group consisting of multiple sclerosis, acute disseminated encephalomyelitis, transverse myelitis, progressive multifocal leukoencephalopathy, subacute sclerosing panencephalitis, chronic inflammatory demyelinating polyneuropathy, and Guillain-Barre syndrome.

4. The quinolone compound for use according to claim 1, wherein the disease induced by deterioration of mitochondrial function is selected from the group consisting of Pearson's syndrome, diabetes, deafness, malignant migraine, Leber's disease, MELAS, MERRF, MERRF/MELAS overlap syndrome, NARP, pure myopathy, mitochondrial cardiomyopathy, myopathy, dementia, gastrointestinal ataxia, acquired sideroblastic anemia, aminoglycoside-induced hearing loss, complex III deficiency due to inherited variants of cytochrome b, multiple symmetric lipomatosis, ataxia, myoclonus, retinopathy, MNGIE, ANT1 disease, Twinkle disease, POLG disease, recurrent myoglobinuria, SANDO, ARCO, complex I deficiency, complex II deficiency, optic nerve atrophy, fatal infantile complex IV deficiency, mitochondrial DNA deficiency syndrome, Leigh's encephalomyelopathy, chronic progressive external ophthalmoplegia syndrome (CPEO), Kearns-Sayre syndrome, encephalopathy, lactacidemia, myoglobinuria, drug-induced mitochondrial diseases, schizophrenia, major depression disorder, bipolar I disorder, bipolar II disorder, mixed episode, dysthymic disorders, atypical depression, seasonal affective disorders, postpartum depression, minor depression, recurrent brief depressive disorder, intractable depression, chronic depression, double depression, and acute renal failure.

## Patentansprüche

1. Chinolonverbindung der folgenden Formel (1) zur Verwendung in der Therapie und/oder Prophylaxe von neurodegenerativen Erkrankungen, durch neurologische Fehlfunktion induzierten Erkrankungen oder durch Verschlechterung der Mitochondrienfunktion induzierten Erkrankungen: 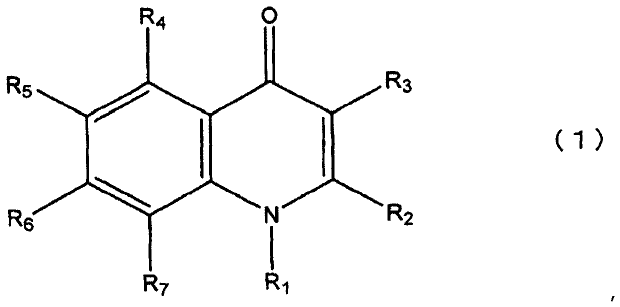 oder ein Salz davon, worin:
R₁ Wasserstoff darstellt;
R₂ Wasserstoff oder C₁₋₆-Alkyl darstellt; und
R₃ Phenyl, Naphthyl, Pyridyl, Furyl, Thienyl, Indolyl,
Benzodioxolyl oder Benzothienyl darstellt, worin der durch R₃ dargestellte aromatische oder heterocyclische Ring mit ein oder mehreren Substituenten ausgewählt aus der Gruppe, die aus den folgenden Substituenten (1) bis (6) besteht, substituiert sein kann:
(1) C₁₋₆-Alkyl,
(2) Halogen-substituiertes C₁₋₆-Alkyl,
(3) C₁₋₆-Alkoxy,
(4) Halogen-substituiertes C₁₋₆-Alkoxy,
(5) Phenyl, das optional ein oder mehrere Substituenten ausgewählt aus der Gruppe bestehend aus C₁₋₆-Alkyl und C₁₋₆-Alkoxy aufweisen kann, und
(6) Halogen;
R₄ Wasserstoff, C₁₋₆-Alkyl, Halogen-substituiertes C₁₋₆-Alkyl, C₁₋₆-Alkoxy, C₁₋₆-Alkoxy-C₁₋₆-alkyl, Phenyl, Cyclo-C₃₋₈-alkyl oder Carbamoyl, das optional ein oder zwei C₁₋₆-Alkylgruppen aufweisen kann, darstellt;
R₅ Wasserstoff, C₁₋₆-Alkyl, Halogen, C₁₋₆-Alkoxy, Benzoylamino oder Imidazolyl darstellt;
R₆ Wasserstoff, Halogen, C₁₋₆-Alkyl oder C₁₋₆-Alkoxy darstellt; und
R₇ eine aus den folgenden Gruppen (1) bis (17) darstellt:
(1) C₁₋₆-Alkyl,
(2) C₁₋₆-Alkoxy,
(3) Phenoxy,
(4) Cyclo-C₃₋₈-alkyloxy,
(5) Halogen,
(6) C₁₋₆-Alkylthio,
(7) Amino, das optional ein oder zwei Substituenten ausgewählt aus der Gruppe bestehend aus C₁₋₆-Alkyl, C₁₋₆-Alkoxy-C₁₋₆-alkyl und Cyclo-C₃₋₈-alkyl aufweist,
(8) Carbamoyl, das optional ein oder zwei C₁₋₆-Alkylgruppen aufweist,
(9) Pyrrolidinyl,
(10) Azepanyl,
(11) Morpholinyl,
(12) Piperazinyl, das optional ein oder zwei C₁₋₆-Alkylgruppen aufweist,
(13) Imidazolyl, das optional ein oder zwei C₁₋₆-Alkylgruppen aufweist,
(14) Furyl,
(15) Thienyl,
(16) Benzothienyl und
(17) Pyrrolidinylcarbonyl.

2. Chinolonverbindung zur Verwendung gemäß Anspruch 1, worin die neurodegenerative Erkrankung aus der Gruppe bestehend aus Parkinson'scher Krankheit, Parkinson'schem Syndrom, juvinilem Parkinsonismus, striatonigraler Degeneration, progressiver supranukleärer Blickparese, reiner Akinese,
Alzheimer'scher Erkrankung, Pick'scher Erkrankung, Prionen-Erkrankung, corticobasaler Degeneration, diffuser Lewis-Körper-Erkrankung, Huntigton'scher Erkrankung, Chorea-Acanthocytosis, gutartiger erblicher Chorea, paroxysmaler Choreoathetose, essentiellem Tremor, essentiellem Myoclonus, Gilles de la Tourette'schem Syndrom, Rett'schem Syndrom, degenerativem Ballismus, Dystonia Musculorum Deformans, Athetosis, Torticolis spasmodicus, Meige-Syndrom, zerebraler Lähmung, Wilson'scher Erkrankung, Segawa'scher Erkrankung, Hallervorden-Spatz-Syndrom, neuroaxonaler Dystrophie, pallidaler Atrophie, spinozerebraler Degeneration, zerebral kortikaler Atrophie, zerebraler Atrophie vom Holmes-Typ, olivopontaozerebraler Atrophie, erblicher olivopontozerebraler Atrophie, Joseph'scher Erkrankung, Dentatorubropallidoluysianischer Atrophie, Gerstmann-Straussler-Scheinker'scher Erkrankung, Friedreich'scher Ataxie, Roussy-Levy-Syndrom, May-White-Syndrom, kongenitaler zerebraler Ataxie, erblicher episodischer Ataxie, Ataxia telangiectasia, amyotropher Lateralsklerose, progressiver bulbarer Lähmung, spinaler progressiver Muskelatrophie, spinobulbarer Muskelatrophie, Werdnig-Hoffmann'scher Erkrankung, Kugelberg-Welander'scher Erkrankung, erblicher spastischer Paraparese, Syringomyelie, Syringobulbie, Arnold-Chiari-Fehlformung, Muskelstarre-Syndrom, Klippel-Feil-Syndrom, Fazio-Londe-Syndrom, unterer Myelopathie, Dandy-Walker-Syndrom, Spina bifida, Sjogren-Larsson-Syndrom, Strahlungs-Myelopathie, altersbedingter Makula-Degeneration und zerebraler Apoplexie ausgewählt aus der Gruppe bestehend aus zerebralem Infarkt und zerebraler Blutung und/oder einer assoziierten Fehlfunktion oder neurologischem Defizit ausgewählt ist.

3. Chinolonverbindung zur Verwendung gemäß Anspruch 1, worin die durch neurologische Fehlfunktion induzierte Erkrankung aus der Gruppe bestehend aus Rückenmarksverletzung, Chemotherapie-induzierte Neuropathie, diabetische Neuropathie, Strahlungsschaden und einer demyelisierenden Erkrankung ausgewählt aus der Gruppe bestehend aus multiple Sklerose, akuter disseminierter Encephalomyelitis, transversaler Myelitis, progressiver multifokaler Leukoencephalopathie, subakuter sklerosierender Panencephalitis, chronisch entzündlicher demyelierender Polyneuropathie und Guillain-Barre-Syndrom ausgewählt ist.

4. Chinolonverbindung zur Verwendung gemäß Anspruch 1, worin die durch Verschlechterung der Mitochondrienfunktion induzierte Erkrankung aus der Gruppe bestehend aus Pearson'schem Syndrom, Diabetes, Taubheit, maligner Migräne, Leber'scher Erkrankung, MELAS, MERRF, MERRF/MELAS-Überlappungssyndrom, NARP, reiner Myopathie, mitochondrialer Kardiomyopathie, Myopathie, Demenz, gastrointestinaler Ataxie, erworbener sideroblastischer Anämie, Aminoglycosidinduziertem Gehörverlust, Komplex III-Defizit aufgrund erblicher Varianten von Cytochrom b, multipler symmetrischer Lipomatose, Ataxie, Myoclonus, Retinopathie, MNGIE, ANT1-Erkrankung, Twinkle-Erkrankung, POLG-Erkrankung, wiederkehrender Myoglobinurie, SANDO, ARCO, Komplex I-Defizit, Komplex II-Defizit, Sehnerv-Atrophie, fatalem infantilen Komplex-Defizit, Mitochondrien-DNA-Defizit-Syndrom, Leigh'scher Encephalomyelopathie, chronisch progressivem externen Ophthalmoplegia-Syndrom (CPEO), Kearns-Sayre-Syndrom, Encephalopathie, Lactacidemie, Myoglobinurie, Arzneimittel-induzierter mitochondrialer Erkrankung, Schizophrenie, schwerer depressiver Störung, bipolarer Störung I, bipolarer Störung II, gemischter Episode, dysthymischen Störungen, atypischer Depression, saisonalen affektiven Störungen, postpartaler Depression, leichter Depression, wiederkehrender kurzer Depressionsstörung, schwer therapierbarer Depression, chronischer Depression, doppelter Depression und akutem Nierenversagen ausgewählt ist.

## Revendications

1. Composé de quinolone représenté par la formule (1) suivante pour son utilisation dans le traitement et/ou la prophylaxie de maladies neurodégénératives, de maladies provoquées par un dysfonctionnement neurologique, ou de maladies provoquées par une détérioration de la fonction mitochondriale : 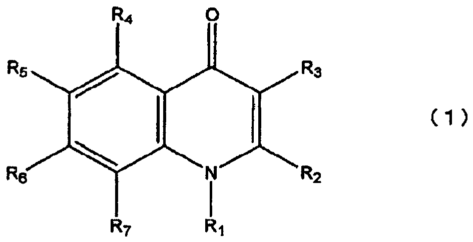 ou un sel de celui-ci, dans laquelle :
R₁ représente un atome d'hydrogène ;
R₂ représente un atome d'hydrogène ou un groupe alkyle en C₁-C₆ ; et
R₃ représente un groupe phényle, un groupe naphtyle, un groupe pyridyle, un groupe furyle, un groupe thiényle, un groupe indolyle, un groupe benzodioxolyle ou un groupe benzothiényle, dans lequel le cycle aromatique ou hétérocyclique représenté par R₃ peut être substitué par un ou plusieurs substituants sélectionnés dans le groupe constitué des substituants (1) à (6) suivants :
(1) un groupe alkyle en C₁-C₆,
(2) un groupe alkyle en C₁-C₆ substitué par un atome d'halogène,
(3) un groupe alcoxy en C₁-C₆,
(4) un groupe alcoxy en C₁-C₆ substitué par un atome d'halogène,
(5) un groupe phényle ayant facultativement un ou plusieurs substituants sélectionnés dans le groupe constitué d'un groupe alkyle en C₁-C₆ et d'un groupe alcoxy en C₁-C₆, et
(6) un atome d'halogène ;
R₄ représente un atome d'hydrogène, un groupe alkyle en C₁-C₆, un groupe alkyle en C₁-C₆ substitué par un atome d'halogène, un groupe alcoxy en C₁-C₆, un groupe (alcoxy en C₁₋C₆) (alkyle en C₁₋C₆), un groupe phényle, un groupe cyclo (alkyle en C₃-C₈), ou un groupe carbamoyle ayant facultativement un ou deux groupes alkyle en C₁-C₆ ;
R₅ représente un atome d'hydrogène, un groupe alkyle en C₁-C₆, un atome d'halogène, un groupe alcoxy en C₁-C₆, un groupe benzoylamino, ou un groupe imidazolyle,
R₆ représente un atome d'hydrogène, un atome d'halogène, un groupe alkyle en C₁-C₆, ou alcoxy en C₁-C₆ ; et
R₇ représente l'un quelconque des groupes suivants (1) à (17) :
(1) un groupe alkyle en C₁-C₆,
(2) un groupe alcoxy en C₁-C₆,
(3) un groupe phénoxy,
(4) un groupe cyclo (alkyloxy en C₃-C₈),
(5) un atome d'halogène,
(6) un groupe alkylthio en C₁-C₆,
(7) un groupe amino ayant facultativement un ou deux substituants sélectionnés dans le groupe constitué d'un groupe alkyle en C₁-C₆, d'un groupe (alcoxy en C₁-C₆) (alkyle en C₁-C₆), et d'un groupe cyclo (alkyle en C₃-C₈),
(8) un groupe carbamoyle ayant facultativement un ou deux groupes alkyle en C₁-C₆,
(9) un groupe pyrrolidinyle,
(10) un groupe azépanyle,
(11) un groupe morpholinyle,
(12) un groupe pipérazinyle ayant facultativement un ou deux groupes alkyle en C₁-C₆,
(13) un groupe imidazolyle ayant facultativement un ou deux groupes alkyle en C₁-C₆,
(14) un groupe furyle,
(15) un groupe thiényle,
(16) un groupe benzothiényle, et
(17) un groupe pyrrolidinylcarbonyle.

2. Composé de quinolone pour son utilisation selon la revendication 1, dans lequel la maladie neurodégénérative est sélectionnée dans le groupe constitué de la maladie de Parkinson, du syndrome de Parkinson, du parkinsonisme juvénile, de la dégénérescence striatonigrale, de la paralysie supranucléaire progressive, de l'akinésie pure, de la maladie d'Alzheimer, de la maladie de Pick, d'une maladie à prions, de la dégénérescence corticobasale, de la maladie à corps de Lewy diffus, de la maladie de Huntington, de la chorée-acanthocytose, de la chorée héréditaire bénigne, de la choréoathétose paroxystique, de tremblement essentiel, de la myoclonie essentielle, du syndrome de Gilles de la Tourette, du syndrome de Rett, du ballisme dégénératif, de la dystonia musculorum deformans, de l'athétose, du torticollis spasmodique, du syndrome de Meige, de l'infirmité motrice cérébrale, de la maladie de Wilson, de la maladie de Segawa, du syndrome de Hallervorden-Spatz, de la dystrophie neuroaxonale, de l'atrophie pallidale, de la dégénérescence spino-cérébelleuse, de l'atrophie cérébro-corticale, de l'atrophie cérébelleuse de type Holmes, de l'atrophie olivopontocérébelleuse, de l'atrophie olivopontocérébelleuse héréditaire, de la maladie de Joseph, de l'atrophie dentato-rubro-pallido-luysiane, de la maladie de Gerstmann-Straussler-Scheinker, de l'ataxie de Friedreich, du syndrome de Roussy-Levy, du syndrome de May-White, de l'ataxie cérébelleuse congénitale, de l'ataxie épisodique héréditaire, de l'ataxie télangiectasie, de la sclérose latérale amyotrophique, de la paralysie bulbaire progressive, de l'atrophie musculaire spinale progressive, de l'atrophie musculaire spinobulbaire, de la maladie de Werdnig-Hoffmann, de la maladie de Kugelberg-Welander, de la paraparésie spastique héréditaire, de la syringomyélie, de la syringobulbie, de la malformation d'Arnold-Chiari, du syndrome de Stiffman, du syndrome de Klippel-Feil, du syndrome de Fazio-Londe, de la myélopathie inférieure, du syndrome de Dandy-Walker, de la spina bifida, du syndrome de Sjogren-Larsson, de la myélopathie post-radique, de la dégénérescence maculaire liée à l'âge, et de l'apoplexie cérébrale sélectionnée dans le groupe constitué d'un infarctus cérébral et d'une hémorragie cérébrale et/ou des dysfonctionnements ou déficits neurologiques associés.

3. Composé de quinolone pour son utilisation selon la revendication 1, dans lequel la maladie provoquée par un dysfonctionnement neurologique est sélectionnée dans le groupe constitué d'un traumatisme médullaire, d'une neuropathie provoquée par la chimiothérapie, de la neuropathie diabétique, d'une radiolésion, et d'une maladie démyélinisante sélectionnée dans le groupe constitué de la sclérose en plaques, de l'encéphalomyélite aiguë disséminée, de la myélite transverse, de la leucoencéphalopathie multifocale progressive, de la panencéphalite sclérosante subaiguë, de la polyneuropathie démyélinisante inflammatoire chronique et du syndrome de Guillain-Barré.

4. Composé de quinolone pour son utilisation selon la revendication 1, dans lequel la maladie provoquée par la détérioration de la fonction mitochondriale est sélectionnée dans le groupe constitué du syndrome de Pearson, du diabète, de la surdité, de la migraine maligne, de la maladie de Leber, du syndrome de MELAS, du syndrome de MERRF, du syndrome de chevauchement MERRF/MELAS, du syndrome NARP, de la myopathie pure, de la cardiomyopathie mitochondriale, de la myopathie, de la démence, de l'ataxie gastro-intestinale, de l'anémie sidéroblastique acquise, de la perte d'audition provoquée par des aminoglycosides, d'un déficit en complexe III du à des variants héréditaires du cytochrome b, de la lipomatose symétrique multiple, de l'ataxie, de la myoclonie, de la rétinopathie, de la MUGIE, de la maladie ANT1, de la maladie de Twinkle, de la maladie de POLG, de la myoglobinurie récurrente, du syndrome SANDO, du syndrome ARCO, d'un déficit en complexe I, d'un déficit en complexe II, d'une atrophie du nerf optique, d'un déficit létal en complexe IV d'apparition infantile, du syndrome de déficit en ADN mitochondrial, de l'encéphalomyélopathie de Leigh, du syndrome d'opthalmoplégie externe chronique progressive (CPEO), du syndrome de Kearns-Sayre, d'une encéphalopathie, de la lactacidémie, de la myoglobinurie, des maladies mitochondriales médicamenteuses, de la schizophrénie, d'un trouble de dépression majeur, d'un trouble bipolaire I, d'un trouble bipolaire II, d'un épisode mixte, des troubles dysthymiques, d'une dépression atypique, de troubles affectifs saisonniers, d'une dépression post-partum, d'une dépression mineure, d'un trouble dépressif bref récurrent, d'une dépression incurable/dépression chronique, d'une double dépression et d'une maladie rénale aiguë.
